# EUROPEAN PATENT APPLICATION

(11) **EP 4 732 759 A1**
(43) Date of publication of application: **29.04.2026**
(21) Application number: 24854280.5
(22) Date of filing: 13.06.2024
(51) Int. Cl.: A61B 5/00, A61B 5/01, A61B 5/11

(54) **WEARABLE DEVICE AND METHOD FOR IDENTIFYING WEARING STATE ON BASIS OF TEMPERATURE, AND NON-TRANSITORY COMPUTER-READABLE RECORDING MEDIUM**

(30) Priority: 11.08.2023 KR 20230105910; 04.09.2023 KR 20230117109
(71) Applicant: Samsung Electronics Co., Ltd., Suwon-si, Gyeonggi-do 16677 (KR)
(72) Inventor: PARK, Hyuncheol, Suwon-si Gyeonggi-do 16677 (KR); KWON, Hyoujoo, Suwon-si Gyeonggi-do 16677 (KR); YOO, Soohan, Suwon-si Gyeonggi-do 16677 (KR); LEE, Seungwon, Suwon-si Gyeonggi-do 16677 (KR); JUNG, Hyunjun, Suwon-si Gyeonggi-do 16677 (KR); JO, Seongwook, Suwon-si Gyeonggi-do 16677 (KR); CHO, Shinhee, Suwon-si Gyeonggi-do 16677 (KR)
(74) Representative: Gulde & Partner
(86) International application number: PCT/KR2024/008160
(87) International publication number: WO 2025/037727

(57) **Abstract**

A wearable device is disclosed. The wearable device may comprise: a temperature sensor arranged to face the skin of a user when the wearable device is worn on the user; at least one processor; and a memory for storing instructions. When executed by the at least one processor, the instructions can cause the wearable device to: identify that the wearable device is worn by the user; and identify, in response to identifying that the wearable device is worn by the user, one wearing state corresponding to temperature data indicating a temperature value measured through the temperature sensor from among a plurality of wearing states.

## Description

### [Technical Field]

The following descriptions relate to a wearable device, a method, and a non-transitory computer readable storage medium that identify a wearing state based on a temperature.

### [Background Art]

A wearable device has changed a way of monitoring the user's health. The wearable device typically worn on a wrist is becoming increasingly popular due to a function capable of tracking various biometric parameters such as a heart rate, a sleep pattern, and an activity level. However, alternative wearable formfactors that may provide an improved function are being required, and a ring-type wearable device with a biometric sensor is being developed to address this requirement.

The above-described information may be provided as a related art for a purpose of helping understanding of the present disclosure. No claim or determination is raised as to whether any of the above-described descriptions may be applied as a prior art related to the present disclosure.

### [Disclosure]

### [Technical Solution]

A wearable device is disclosed. The wearable device may include a temperature sensor arranged to face skin of a user, when the wearable device is worn by the user. The wearable device may include at least one processor. The wearable device may include memory storing instructions. The instructions may be configured, when executed by the at least one processor, to cause the wearable device to identify that the wearable device is worn by the user. The instructions may be configured, when executed by the at least one processor, to cause the wearable device to, in response to identifying that the wearable device is worn by the user, identify one wearing state corresponding to temperature data among a plurality of wearing states, the temperature data indicating a temperature value measured through the temperature sensor.

A method is disclosed. The method may be performed by the wearable device including a temperature sensor arranged to face skin of a user, when the wearable device is worn by the user. The method may include identifying that the wearable device is worn by the user. The method may include, in response to identifying that the wearable device is worn by the user, identifying one wearing state corresponding to temperature data among a plurality of wearing states, the temperature data indicating a temperature value measured through the temperature sensor.

A non-transitory computer readable storage medium is disclosed. The non-transitory computer readable storage medium may store a program including instructions. The instructions may be configured, when executed by at least one processor of a wearable device including a temperature sensor arranged to face skin of a user when the wearable device is worn by the user, to cause the wearable device to identify that the wearable device is worn by the user. The instructions may be configured, when executed by the at least one processor, to cause the wearable device to, in response to identifying that the wearable device is worn by the user, identify one wearing state corresponding to temperature data among a plurality of wearing states, the temperature data indicating a temperature value measured through the temperature sensor.

### [Description of the Drawings]

FIG. 1 illustrates an exemplary wearable device according to an embodiment.
FIG. 2 indicates a cross-section of an exemplary wearable device according to an embodiment.
FIG. 3 is a block diagram of a wearable device according to an embodiment.
FIG. 4 illustrates an example of a state diagram of a wearable device according to an embodiment.
FIG. 5 illustrates an example of a graph indicating a temperature measured by a wearable device according to an embodiment.
FIG. 6A illustrates an example of wearing states of a wearable device according to an embodiment.
FIG. 6B illustrates an example of a graph indicating a temperature in wearing states of a wearable device according to an embodiment.
FIG. 7 illustrates an example of providing a notification to a user by a wearable device according to an embodiment.
FIG. 8A exemplifies a location at which a wearable device is worn, according to an embodiment.
FIG. 8B exemplifies biometric data obtained by a wearable device according to an embodiment.
FIG. 9 is a flowchart indicating an operation of a wearable device according to an embodiment.
FIG. 10 is a flowchart indicating an operation of a wearable device according to an embodiment.
FIG. 11 is a flowchart indicating an operation of a wearable device according to an embodiment.
FIG. 12 is a flowchart indicating an operation of a wearable device according to an embodiment.
FIG. 13 is a flowchart indicating an operation of a wearable device according to an embodiment.
FIG. 14 is a block diagram of an electronic device in a network environment according to various embodiments.

### [Mode for Invention]

Hereinafter, an embodiment of the present disclosure will be described in detail with reference to the drawings so that those having ordinary knowledge in the art to which the present disclosure belongs may easily implement it. However, the present disclosure may be implemented in several different forms and is not limited to the embodiment described herein. In association with a description of the drawings, the same or similar reference numerals may be used for the same or similar components. In addition, in the drawings and the related description, a description of well-known functions and configurations may be omitted for clarity and brevity.

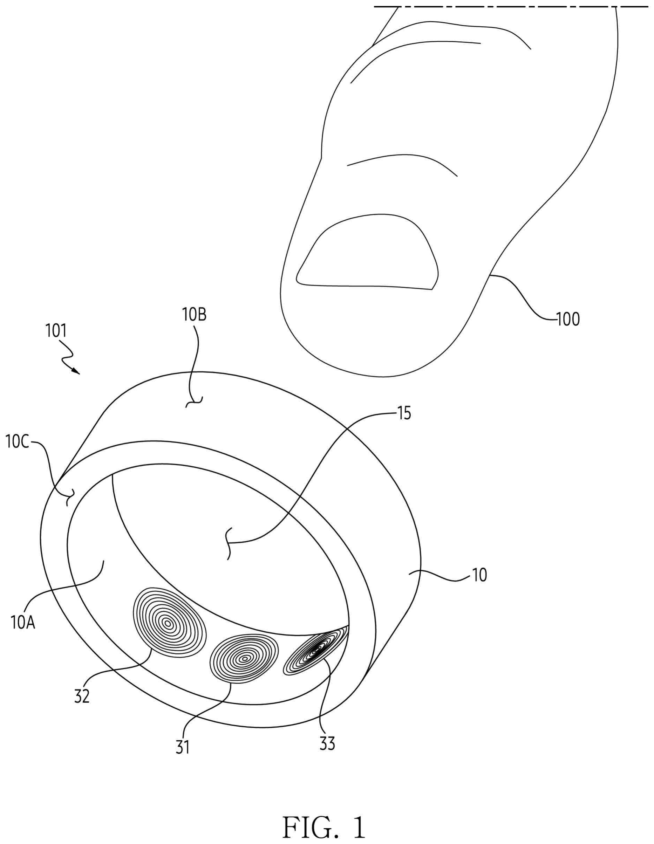

FIG. 1 illustrates an exemplary wearable device according to an embodiment.

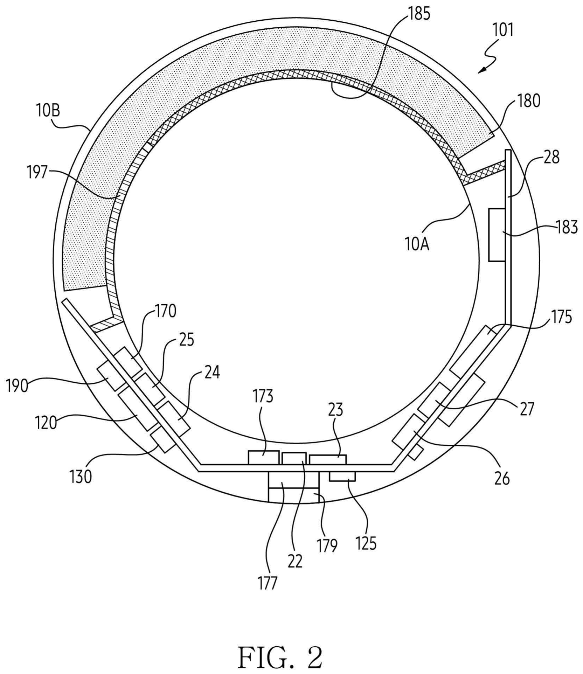

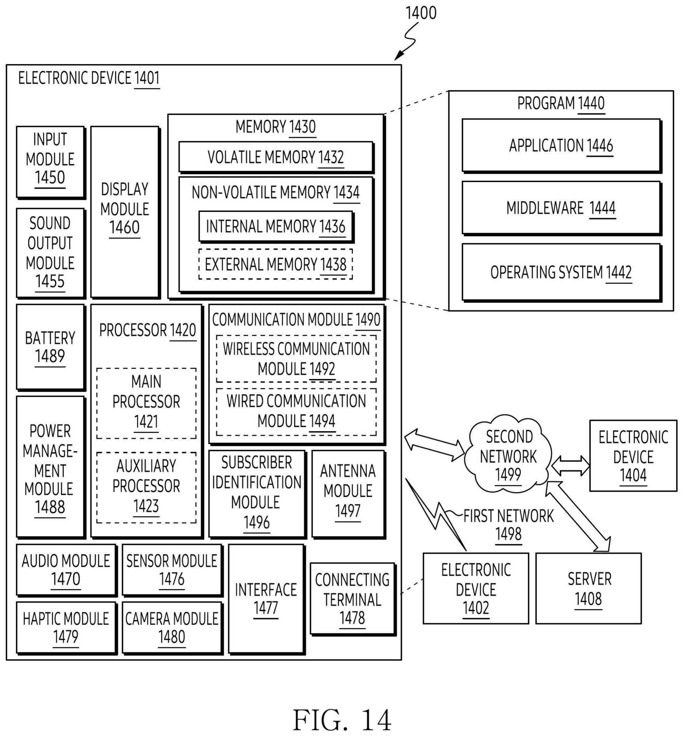

Referring to FIGS. 1 and 2, a wearable device 101 (e.g., an electronic device 1401 of FIG. 14) according to an embodiment may be configured to be wearable by a user. For example, the wearable device 101 may have a ring shape with a hole 15 such that the user may insert a body (or a part of the body) 100 (e.g., a finger). However, it is not limited thereto, and the wearable device 101 may have various shapes corresponding to the body in order to be worn by the body of the user.

In an embodiment, the wearable device 101 may include a housing 10.

Referring to FIG. 1, the housing 10 may form an exterior of the wearable device 101. For example, the housing 10 may form or define a first surface 10A, a second surface 10B, and a third surface 10C. When the user wears the wearable device 101, the first surface 10A may surround the body of the user to face the body 100 of the user. The first surface 10A may at least partially contact the body 100 of the user. For example, a first region 31, a second region 32, and a third region 33 formed on the first surface 10A may be in contact with the body 100 of the user. The first region 31, the second region 32, and the third region 33 may be spaced apart from each other. The first region 31 may be located between the second region 32 and the third region 33. The second surface 10B may be spaced apart from the first surface 10A and may face an opposite direction to the first surface 10A. The third surface 10C may surround a space between the first surface 10A and the second surface 10B. For example, the third surface 10C may extend from a periphery of the first surface 10A to a periphery of the second surface 10B. The hole 15 defined by the first surface 10A may be formed in the housing 10 to accommodate the body 100 of the user. The first surface 10A may be referred to as an inner circumferential surface, and the second surface 10B may be referred to as an outer circumferential surface.

In an embodiment, at least a portion of the first surface 10A of the housing 10 may be formed by an insulating member. For example, the insulating member may form at least the first region 31, the second region 32, and the third region 33 of the first surface 10A. For example, the insulating member may form the first region 31, the second region 32, and the third region 33, and may also form a portion of the first surface 10A extending from the first region 31, the second region 32, and the third region 33. The first to third regions 31, 32, and 33 may be referred to as first to third sensing regions, first to third pattern regions, first to third Fresnel pattern regions, or first to third condensing regions, respectively.

In an embodiment, the second surface 10B and the third surface 10C of the housing 10 may be formed by a frame, but are not limited thereto. For example, the second surface 10B and/or the third surface 10C may also be formed by the frame and the insulating member together.

In an embodiment, a frame of the housing 10 may be formed of metal and/or plastic. The frame and the insulating member may be coupled to each other. For example, through a process such as insert injection, the insulating member and the frame may be coupled. For example, an insulating member coupled to the frame may be formed by injecting a molten resin into a mold in which the frame is disposed, but is not limited thereto. The insulating member may include, for example, a resin such as epoxy, but is not limited thereto.

FIG. 2 indicates a cross-section of an exemplary wearable device according to an embodiment.

Referring to FIG. 2, a wearable device 101 may include at least one light emitting unit 22, 24, and/or 26 and at least one light receiving unit 23, 25, and/or 27. In an embodiment, the wearable device 101 may include a substrate 28. In an embodiment, the wearable device 101 may include a processor 120, a controller 125, memory 130, a temperature sensor 170, a motion sensor 173, a pressure sensor 175, an external temperature sensor 177, a lens 179, a battery 180, a power management module 183, a charging interface 185, communication circuitry 190, and an antenna 197.

In an embodiment, the substrate 28 may include a flexible printed circuit board or a hard-flexible printed circuit board. In an embodiment, the substrate 28 may be at least partially curved. For example, the substrate 28 may include a bent portion to correspond to a curvature of a first surface 10A having a ring shape.

In an embodiment, the processor 120 may correspond to a processor 1420 of FIG. 14. In an embodiment, the controller 125 may be included in the processor 1420 of FIG. 14. In an embodiment, the controller 125 may be included in a sensor module 1476 of FIG. 14. In an embodiment, the memory 130 may correspond to memory 1430 of FIG. 14. In an embodiment, each of the at least one light emitting unit 22, 24, and/or 26, the at least one light receiving unit 23, 25, and/or 27, the temperature sensor 170, the motion sensor 173, the pressure sensor 175, and the external temperature sensor 177 may correspond to the sensor module 1476 of FIG. 14. In an embodiment, the battery 180 may correspond to a battery 1489 of FIG. 14. In an embodiment, the power management module 183 may correspond to a power management module 1488 of FIG. 14. In an embodiment, the communication circuitry 190 may correspond to a communication module 1490 of FIG. 14. In an embodiment, the antenna 197 may correspond to an antenna module 1497 of FIG. 14. In an embodiment, the charging interface 185 may include wired and/or wireless interface circuitry for receiving power from an external power source for charging the battery 180.

In an embodiment, the at least one light emitting unit 22, 24, and/or 26 and the at least one light receiving unit 23, 25, and/or 27 may also be referred to as a photoplethysmography (PPG) sensor. However, it is not limited thereto. In an embodiment, the at least one light emitting unit 22, 24, and/or 26 and the at least one light receiving unit 23, 25, and/or 27 may also be referred to as a proximity sensor.

In an embodiment, the at least one light emitting unit 22, 24, and/or 26 and the at least one light receiving unit 23, 25, and/or 27 may be disposed on the substrate 28. For example, the at least one light emitting unit 22, 24, and/or 26 may be disposed on the substrate 28 to face the first surface 10A. For example, the at least one light receiving unit 23, 25, and/or 27 may be disposed on the substrate 28 to face the first surface 10A.

In an embodiment, the light emitting unit 22 and the light receiving unit 23 may face a first region 31 of an insulating member. The light emitting unit 22 and the light receiving unit 23 may be aligned with the first region 31 of the insulating member. The light emitting unit 22 and the light receiving unit 23 may overlap the first region 31. In an embodiment, the light emitting unit 24 and the light receiving unit 25 may face a second region 32 of the insulating member. The light emitting unit 24 and the light receiving unit 25 may be aligned with the second region 32 of the insulating member. The light emitting unit 24 and the light receiving unit 25 may overlap the second region 32. In an embodiment, the light emitting unit 26 and the light receiving unit 27 may face a third region 33 of the insulating member. The light emitting unit 26 and the light receiving unit 27 may be aligned with the third region 33 of the insulating member. The light emitting unit 26 and the light receiving unit 27 may overlap the third region 33.

In an embodiment, the at least one light emitting unit 22, 24, and/or 26 may be configured to emit light toward a body 100 of a user. In an embodiment, the at least one light emitting unit 22, 24, and/or 26 may be configured to emit light toward the body 100 of the user under control of the controller 125. The light emitted from the at least one light emitting unit 22, 24, and/or 26 may pass through the insulating member and be transmitted to the body 100 of the user. For example, the light emitted from the light emitting unit 22 may be transmitted to the body 100 of the user through the first region 31. For example, the light emitted from the light emitting unit 24 may be transmitted to the body 100 of the user through the second region 32. For example, the light emitted from the light emitting unit 26 may be transmitted to the body 100 of the user through the third region 33. The light from the at least one light emitting unit 22, 24, and/or 26 may be reflected from the body 100 of the user. The light reflected from the body 100 of the user may reach the at least one light receiving unit 23, 25, and/or 27 by passing through the insulating member. The light reflected from the body 100 of the user may reach the at least one light receiving unit 23, 25, and/or 27 through at least one region 31, 32, and/or 33. The at least one light receiving unit 23, 25, and/or 27 may receive light incident from the outside through the insulating member. In an embodiment, the at least one light receiving unit 23, 25, and/or 27 may be configured to receive light from the body 100 of the user under the control of the controller 125. However, it is not limited thereto. For example, the light from the at least one light emitting unit 22, 24, and/or 26 may penetrate (or pass through) the body 100 of the user. The light penetrated (or passed through) the body 100 of the user may reach the at least one light receiving unit 23, 25, and/or 27 through the at least one region 31, 32, and/or 33.

In an embodiment, the motion sensor 173 may include an acceleration sensor and/or a gyro sensor. For example, the motion sensor 173 may be a three-axis sensor (e.g., the acceleration sensor). For example, the motion sensor 173 may be a six-axis sensor (e.g., the acceleration sensor and the gyro sensor).

In an embodiment, the pressure sensor 175 may be disposed on the substrate 28 to face the first surface 10A. In an embodiment, the pressure sensor 175 may measure a pressure value applied to at least a portion of the first surface 10A.

In an embodiment, the external temperature sensor 177 may be disposed on the substrate 28 to face a second surface 10B. The external temperature sensor 177 may measure a temperature of a temperature measurement object based on infrared rays emitted by the temperature measurement object. In an embodiment, the lens 179 for penetrating the infrared rays may be provided such that the infrared rays emitted by the temperature measurement object may be received by the external temperature sensor 177. The lens 179 may be disposed to face the second surface 10B.

In an embodiment, the processor 120 may obtain biometric information of the user using a sensor module. The processor 120 may detect the biometric information of the user based on the light emitted from the at least one light emitting unit 22, 24, and/or 26 and the light received by the at least one light receiving unit 23, 25, and/or 27. For example, the biometric information may include information on a heart rate and/or saturation of percutaneous oxygen (SpO2), but is not limited thereto. For example, the biometric information may include information on arterial stiffness, blood pressure, or an arterial age. Herein, the heart rate may indicate the number of heartbeats per unit time. The saturation of percutaneous oxygen may indicate a ratio of an amount of hemoglobin coupled with oxygen in a blood to a total amount of hemoglobin. The arterial stiffness may indicate a degree to which a blood vessel is hardened. The blood pressure may indicate pressure applied to an arterial wall when blood sent from a heart flows through the blood vessel. The arterial age, which is a physiological age indicating a degree of aging of the blood vessel, may be related to the arterial stiffness. The biometric information may include information on stress, blood sugar, and/or irregular heart rhythm notification (IHRN).

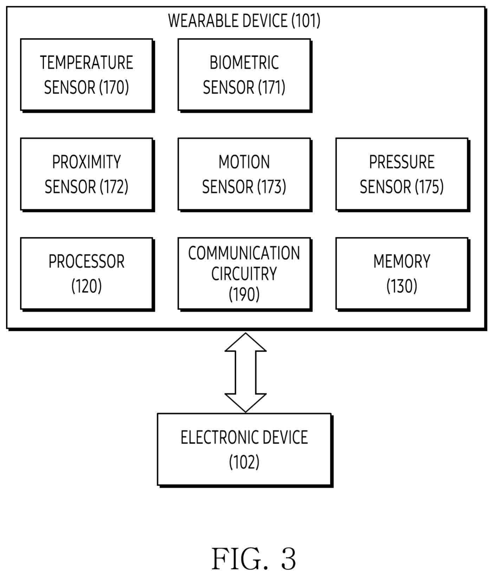

FIG. 3 is a block diagram of a wearable device 101.

Referring to FIG. 3, the wearable device 101 may include a processor 120, memory 130, a temperature sensor 170, a biometric sensor 171, a proximity sensor 172, a motion sensor 173, a pressure sensor 175, and communication circuitry 190.

The wearable device 101 may correspond to an electronic device 1401 of FIG. 14. However, it is not limited thereto. For example, the wearable device 101 may correspond to an electronic device 1402 of FIG. 14. The processor 120 of FIG. 3 may correspond to the processor 120 of FIG. 2. The memory 130 of FIG. 3 may correspond to the memory 130 of FIG. 2. The temperature sensor 170 of FIG. 3 may correspond to the temperature sensor 170 of FIG. 2. The biometric sensor 171 of FIG. 3 may correspond to the at least one light emitting unit 22, 24, and/or 26 and the at least one light receiving unit 23, 25, and/or 27 of FIG. 2. The motion sensor 173 of FIG. 3 may correspond to the motion sensor 173 of FIG. 2. The pressure sensor 175 of FIG. 3 may correspond to the pressure sensor 175 of FIG. 2. The communication circuitry 190 of FIG. 3 may correspond to the communication circuitry 190 of FIG. 2.

The proximity sensor 172 may be a sensor for emitting infrared light and obtaining reflected light of a finger of the infrared light. For example, the proximity sensor 172 may include the at least one light emitting unit 22, 24, and/or 26 and the at least one light receiving unit 23, 25, and/or 27. However, it is not limited thereto. The proximity sensor 172 may be an electrode and/or a touch sensor. For example, the proximity sensor 172 may detect whether a body 100 is in contact with the electrode and/or the touch sensor.

In an embodiment, the processor 120 may establish a communication connection with an electronic device 102 through the communication circuitry 190. Herein, the electronic device 102 may be a device of various forms. For example, the electronic device 102 may be a portable communication device (e.g., a smartphone), a computer device, a portable multimedia device, a portable medical device, a camera, a wearable device (e.g., a watch), or a home appliance. The electronic device 102 may correspond to the electronic device 1402 of FIG. 14. However, it is not limited thereto. For example, the electronic device 102 may correspond to the electronic device 1401 of FIG. 14.

Hereinafter, an operation while the wearable device 101 is not worn by the body 100 of the user will be described.

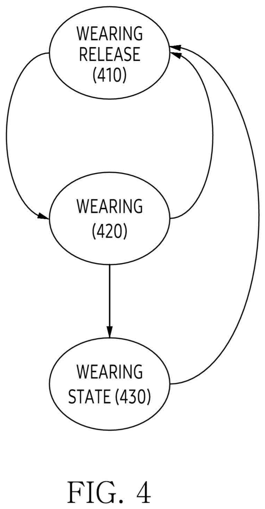

FIG. 4 illustrates an example of a state diagram of a wearable device 101. Referring to FIG. 4, a state of the wearable device 101 may transition between a wearing release 410, wearing 420, and a wearing state 430. Herein, the wearing release 410 may mean a state in which wearing of the wearable device 101 is released from a user. The wearing 420 may mean a state in which the wearable device 101 is worn by the user. The wearing state 430 may indicate how loosely (or tightly) the wearable device 101 is worn by the user.

A processor 120 may operate in a low-power mode (or a sleep mode) while the state of the wearable device 101 is the wearing release 410. Herein, the low-power mode (or the sleep mode) may mean turning off other functions other than a designated function. The low-power mode (or the sleep mode) may mean that the other functions other than the designated function are limited. The low-power mode (or the sleep mode) may mean turning off other components other than a designated component. The low-power mode (or the sleep mode) may mean that power consumption of the other components other than the designated component is limited. For example, an operating frequency of the processor 120 may be decreased during the low-power mode (or the sleep mode). For example, other sensors (e.g., a temperature sensor 170, a biometric sensor 171, a proximity sensor 172, and a pressure sensor 175) other than a motion sensor 173 may be turned off during the low-power mode (or the sleep mode). However, it is not limited thereto.

The processor 120 may obtain movement data indicating movement of the wearable device 101 through the motion sensor 173. For example, the processor 120 may obtain movement data indicating three-axis movement (e.g., a change in acceleration in a space or a change in angular velocity in a space). For example, the processor 120 may obtain movement data indicating six-axis movement (e.g., a change in acceleration in a space or a change in angular velocity in a space).

The processor 120 may determine whether the wearable device 101 moves based on the movement data. The processor 120 may determine whether the wearable device 101 moves more than or equal to a designated distance (e.g., 50 cm) based on the movement data. In a case that the wearable device 101 moves more than or equal to the designated distance, the processor 120 may determine that the wearable device 101 has moved. In a case that the wearable device 101 moves less than the designated distance, the processor 120 may determine that the wearable device 101 has not moved.

The processor 120 may determine whether the wearable device 101 is worn by the user based on the movement data indicating the movement of the wearable device 101. The processor 120 may identify whether the wearable device 101 is worn, in response to the movement data indicating the movement of the wearable device 101. For example, in a case that the wearable device 101 is identified as being moved more than or equal to the designated distance based on the movement data, the processor 120 may identify whether the wearable device 101 is worn by the user. Herein, whether the wearable device 101 is worn may be identified by another sensor (e.g., the temperature sensor 170 and/or the proximity sensor 172).

The processor 120 may release the low-power mode (or the sleep mode) based on the movement data indicating the movement of the wearable device 101. The processor 120 may transition from the low-power mode (or the sleep mode) to a normal mode based on the movement data indicating the movement of the wearable device 101. For example, in a case that the wearable device 101 is identified as being moved more than or equal to the designated distance based on the movement data, the processor 120 may release the low-power mode (or the sleep mode). Herein, releasing the low-power mode (or the sleep mode) may mean that all functions operate normally. Releasing the low-power mode (or the sleep mode) may mean that all components operate normally.

Hereinafter, an operation of identifying that the wearable device 101 is worn by a body 100 of the user will be described.

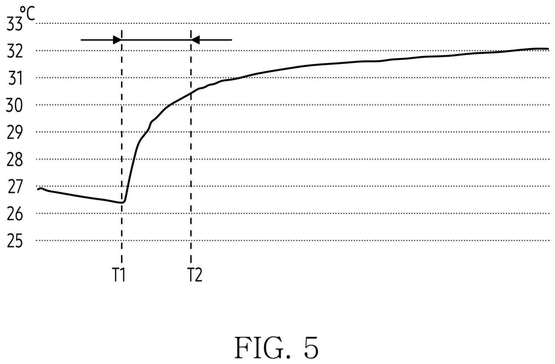

FIG. 5 illustrates an example of a graph indicating a temperature measured by a wearable device 101 according to an embodiment.

In an embodiment, a processor 120 may identify that the wearable device 101 is worn by a user. In an embodiment, the processor 120 may identify that the wearable device 101 is worn by the user, based on a temperature sensor 170 and/or a proximity sensor 172. The processor 120 may identify that the wearable device 101 is worn by the user, while a state of the wearable device 101 is a wearing release 410.

In an embodiment, the processor 120 may identify that the wearable device 101 is worn by the user, based on the temperature sensor 170. In an embodiment, the processor 120 may identify that the wearable device 101 is worn by the user, based on a temperature value obtained through the temperature sensor 170. For example, the processor 120 may identify that the wearable device 101 is worn by the user, based on whether the temperature value changes more than or equal to a reference temperature value (e.g., 4 degrees). For example, the processor 120 may identify that the wearable device 101 is worn by the user, based on whether the temperature value changes more than or equal to the reference temperature value within a designated time period (e.g., 10 seconds). For example, in a case that the temperature value changes more than or equal to the reference temperature value (e.g., 4 degrees) during a specific time period (e.g., a time period of 10 seconds), the processor 120 may identify that the wearable device 101 is worn by the user. However, 10 seconds and 4 degrees are merely an example, and are not limited thereto.

For example, as illustrated in FIG. 5, in a case that a difference between a temperature at a time point T1 and a temperature at a time point T2 approximately 10 seconds after the time point T1 exceeds the reference temperature value (e.g., 4 degrees), the processor 120 may identify that the wearable device 101 is worn by the user after the time point T1.

In an embodiment, the processor 120 may identify that the wearable device 101 is worn by the user, based on the proximity sensor 172. In an embodiment, the processor 120 may identify that the wearable device 101 is worn by the user, based on an intensity value indicating intensity of light obtained through the proximity sensor 172. For example, the processor 120 may identify that the wearable device 101 is worn by the user based on whether the intensity value is more than or equal to a reference intensity value. For example, in a case that the intensity value is more than or equal to the reference intensity value, the processor 120 may identify that the wearable device 101 is worn by the user.

In an embodiment, the processor 120 may identify that the wearable device 101 is worn by the user, based on the temperature sensor 170 and the proximity sensor 172. The processor 120 may identify a first state of the wearable device 101 based on the temperature sensor 170. The processor 120 may identify a second state of the wearable device 101 based on the proximity sensor 172. In a case that the first state indicates that the wearable device 101 is worn by the user, and the second state indicates that the wearable device 101 is worn by the user, the processor 120 may identify that the wearable device 101 is worn by the user. In a case that at least one state among the first state or the second state indicates that the wearable device 101 is not worn by the user, the processor 120 may identify that the wearable device 101 is not worn by the user.

In an embodiment, when identifying that the wearable device 101 is worn by the user, the state of the wearable device 101 may transition from the wearing release 410 to wearing 420.

Hereinafter, an operation in which the wearable device 101 identifies a wearing state will be described.

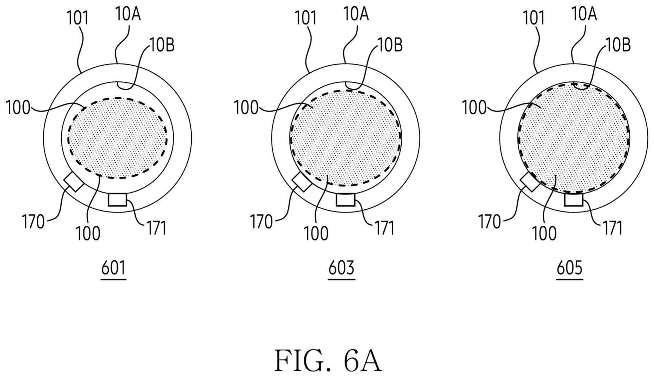

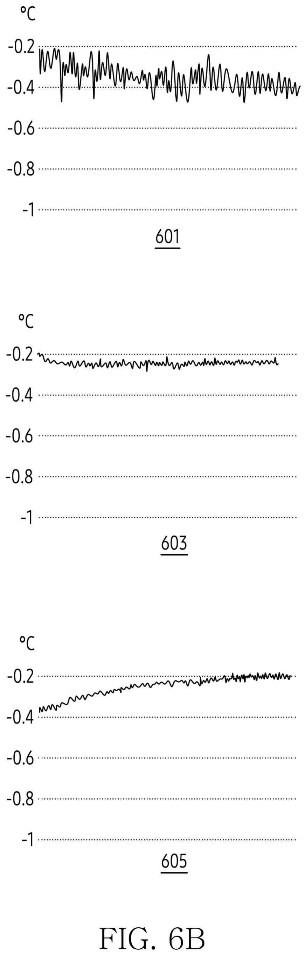

FIG. 6A illustrates an example of wearing states of a wearable device according to an embodiment. FIG. 6B illustrates an example of a graph indicating a temperature in wearing states of a wearable device according to an embodiment.

In an embodiment, a processor 120 may identify a wearing state of a wearable device 101. In response to identifying that the wearable device 101 is worn by a user, the processor 120 may identify the wearing state of the wearable device 101. Herein, the wearing state may be configured with a plurality of wearing states. The plurality of wearing states may indicate different degrees of loosening (or degrees of tightness) between the wearable device 101 and a body 100. The processor 120 may identify the wearing state of the wearable device 101 while a state of the wearable device 101 is wearing 420.

For example, a plurality of wearing states 601, 603, and 605 may correspond to a plurality of finger circumferences. For example, the plurality of wearing states 601, 603, and 605 may correspond to a state of being worn by each of fingers having a finger circumference of a designated range (e.g., 44 millimeters to 69 millimeters). For example, the plurality of wearing states 601, 603, and 605 may be set to have 1 millimeter interval in a range of approximately 44 millimeters to approximately 69 millimeters. For example, a first wearing state may correspond to a state of being worn by a finger of approximately 44 millimeters. For example, a second wearing state may correspond to a state of being worn by a finger of approximately 45 millimeters. For example, each of 24th, 25th, and 26th wearing states may correspond to a state of being worn by each of fingers of 67, 68, and 69 millimeters. However, it is not limited thereto.

For example, the plurality of wearing states 601, 603, and 605 may be divided into N wearing states. For example, the plurality of wearing states 601, 603, and 605 may include N-2 wearing states between the tight state 605 and the loose state 601. For example, in a case that the N is 3, the plurality of wearing states 601, 603, and 605 may be divided into three wearing states. For example, the plurality of wearing states 601, 603, and 605 may be divided into the tight state 605, the normal state 603, or the loose state 601. In this case, in a case of being worn by a finger having finger circumferences of a portion (e.g., approximately 44 millimeters to approximately 49 millimeters) of finger circumferences of the designated range (e.g., 44 millimeters to 69 millimeters), the processor 120 may divide the wearing state of the wearable device 101 into the loose state 601. In a case of being worn by a finger having finger circumferences of a portion (e.g., approximately 50 millimeters to approximately 59 millimeters) of finger circumferences of the designated range (e.g., 44 millimeters to 69 millimeters), the processor 120 may divide the wearing state of the wearable device 101 into the normal state 603. In a case of being worn by a finger having finger circumferences of a portion (e.g., approximately 60 millimeters to approximately 69 millimeters) of finger circumferences of the designated range (e.g., 44 millimeters to 69 millimeters), the processor 120 may divide the wearing state of the wearable device 101 into the tight state 605. However, it is not limited thereto. Hereinafter, it may be described that the plurality of wearing states are three wearing states.

The processor 120 may identify the wearing state of the wearable device 101 based on a temperature sensor 170. The processor 120 may identify one wearing state corresponding to temperature data indicating a temperature value measured through the temperature sensor 170 among the plurality of wearing states. For example, the processor 120 may identify one wearing state corresponding to a change in a temperature value indicated by temperature data among the plurality of wearing states 601, 603, and 605. For example, the processor 120 may identify one wearing state corresponding to a change in a temperature value indicated by temperature data obtained for a designated time period. For example, the processor 120 may identify one wearing state corresponding to variance, deviation, and/or standard deviation of the temperature value indicated by the temperature data obtained for the designated time period. For example, in a case that a range for each of the plurality of wearing states is set, the processor 120 may identify a wearing state of a range to which a value (e.g., variance, deviation, and/or standard deviation) indicating a change in a temperature value belongs as the wearing state of the wearable device 101.

For example, referring to FIG. 6B, a variation range of a temperature in the loose state 601 may be greater than a variation range of a temperature in the tight state 605. A variation range of a temperature in the normal state 603 may be located between a variation range of a temperature in the loose state 601 and a variation range of a temperature in the tight state 605. In addition, standard deviation of the temperature in the loose state 601 may be greater than standard deviation of the temperature in the tight state 605. Standard deviation of the temperature in the normal state 603 may be located between standard deviation of the temperature in the loose state 601 and standard deviation of the temperature in the tight state 605. For example, in a case that the standard deviation of each of the loose state 601, the normal state 603, or the tight state 605 is approximately 0.032, approximately 0.051, or approximately 0.084, standard deviation of a temperature to divide the loose state 601, the normal state 603, or the tight state 605 may be determined by a value (e.g., approximately 0.042) between approximately 0.032 and approximately 0.051 and a value (e.g., approximately 0.068) between approximately 0.051 and approximately 0.084. For example, the processor 120 may determine the state of the wearable device 101 as one of the loose state 601, the normal state 603, or the tight state 605 according to which section among three sections where the standard deviation of the temperature value indicated by the temperature data obtained for the designated time period is divided into approximately 0.042 and approximately 0.068.

In an embodiment, when the wearing state of the wearable device 101 is identified, the state of the wearable device 101 may transition from the wearing 420 to the wearing state 430.

Hereinafter, an operation in which the wearable device 101 provides a notification to the user based on the wearing state will be described.

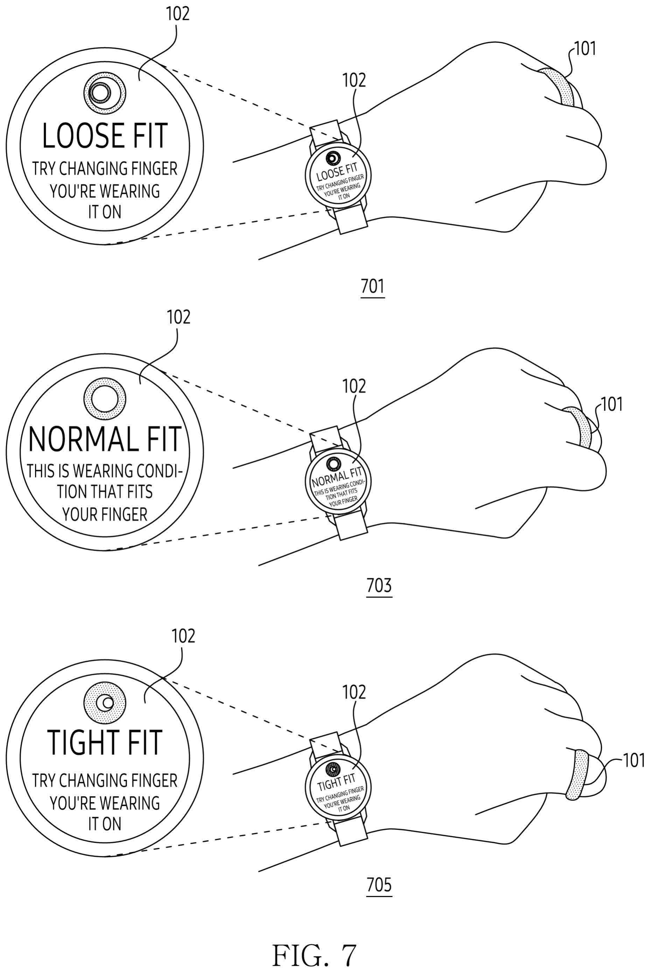

FIG. 7 illustrates an example of providing a notification to a user by a wearable device according to an embodiment.

In an embodiment, a processor 120 may identify a wearing state. The processor 120 may identify the wearing state based on temperature data indicating a temperature value measured through a temperature sensor 170. For example, the processor 120 may identify the wearing state based on a change in a temperature value indicated by temperature data obtained for a designated time period. For example, the processor 120 may identify the wearing state based on variance, deviation, and/or standard deviation of the temperature value indicated by the temperature data obtained for the designated time period.

In an embodiment, the processor 120 may output a notification for indicating the identified wearing state. The processor 120 may enable an electronic device 102 to output the notification indicating the wearing state. For example, the processor 120 may transmit data indicating the identified wearing state to the electronic device 102 based on a communication connection with the electronic device 102. The electronic device 102 may output the notification indicating the wearing state based on the data indicating the identified wearing state received from the wearable device 101. However, it is not limited thereto. The processor 120 may output the notification indicating the wearing state through the wearable device 101. For example, the processor 120 may output the notification indicating the wearing state through a component (e.g., a sound output module 1455, a display module 1460, and/or a haptic module 1479 of FIG. 14), which may interact with a user, included in the wearable device 101. Herein, the notification indicating the wearing state may include information (e.g., "Try changing the finger you're wearing it on" and "This is a wearing condition that fits your finger") indicating whether the wearing state is suitable. However, it is not limited thereto.

For example, referring to FIG. 7, in a loose wearing state 701, the wearable device 101 may provide a notification (e.g., "Loose fit" and/or "Try changing the finger you're wearing it on") notifying that it is in a loose wearing state to the user through the electronic device 102. In a normal wearing state 703, the wearable device 101 may provide a notification (e.g., "Normal fit" and/or "This is a wearing condition that fits your finger") notifying that it is in a normal wearing state to the user through the electronic device 102. In a tight wearing state 705, the wearable device 101 may provide a notification (e.g., "Tight fit" and/or "Try changing the finger you're wearing it on") notifying that it is in a tight wearing state to the user through the electronic device 102.

Hereinafter, an operation in which the wearable device 101 provides a notification to the user based on a change in the wearing state will be described.

In an embodiment, the processor 120 may identify a change in the wearing state. For example, the processor 120 may identify the change in the wearing state while the wearable device 101 is worn by the user. The processor 120 may identify the change in the wearing state based on temperature data indicating a temperature value measured through the temperature sensor 170. For example, the processor 120 may identify the change in the wearing state based on a change in a temperature value indicated by temperature data obtained for a designated time period. For example, the processor 120 may identify the change in the wearing state based on variance, deviation, and/or standard deviation of the temperature value indicated by the temperature data obtained for the designated time period.

In an embodiment, the processor 120 may identify whether the changed wearing state is a designated wearing state. Herein, the designated wearing state may be a tight wearing state.

In an embodiment, the processor 120 may output a notification to guide the change in the wearing state based on identifying that the changed wearing state is the designated wearing state.

In an embodiment, the processor 120 may output the notification based on the changed wearing state being maintained in the designated wearing state for more than or equal to the designated time period. For example, the processor 120 may output the notification based on the wearing state of the wearable device 101 being maintained in the designated wearing state for more than or equal to the designated time period after the changed wearing state is identified as the designated wearing state. Herein, the designated time period may be set to be shorter than a time period when the user feels uncomfortable due to wearing of the wearable device 101. The time period when the user feels uncomfortable due to the wearing of the wearable device 101 may be measured experimentally.

In an embodiment, the processor 120 may determine whether to output a notification to guide the change in the wearing state through a pressure sensor 175 based on identifying that the changed wearing state is the designated wearing state. For example, the processor 120 may output the notification based on a pressure value indicated by a pressure signal obtained through the pressure sensor 175 in response to identifying that the changed wearing state is the designated wearing state. For example, in response to identifying that the changed wearing state is the designated wearing state, the processor 120 may output the notification based on that the pressure value indicated by the pressure signal obtained through the pressure sensor 175 exceeds a designated pressure value.

In an embodiment, the processor 120 may output the notification to guide the change in the wearing state through the electronic device 102. The processor 120 may enable the electronic device 102 to output the notification to guide the change in the wearing state. For example, the processor 120 may transmit data indicating the identified wearing state to the electronic device 102 based on the communication connection with the electronic device 102. The electronic device 102 may output the notification to guide the change in the wearing state based on data indicating the identified wearing state received from the wearable device 101. However, it is not limited thereto. The processor 120 may output the notification to guide the change in the wearing state through the wearable device 101. For example, the processor 120 may output the notification to guide the change in the wearing state through the component (e.g., the sound output module 1455, the display module 1460, and/or the haptic module 1479 of FIG. 14), which may interact with the user, included in the wearable device 101 and . Herein, the notification to guide the change in the wearing state may include information (e.g., "Change to the fourth finger") indicating a location of a suitable finger for the user. However, it is not limited thereto.

Hereinafter, an operation in which the wearable device 101 obtains biometric information of the user based on the wearing state will be described.

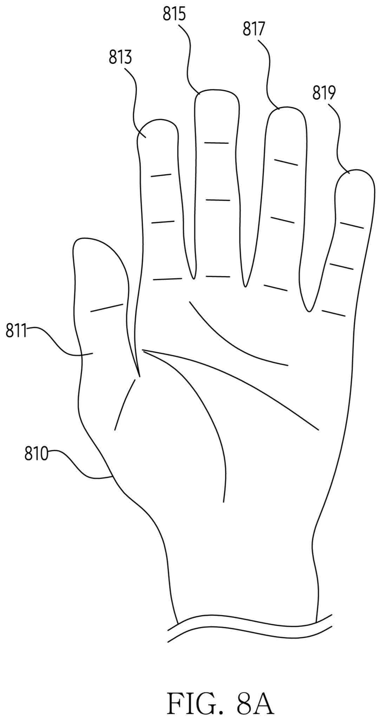

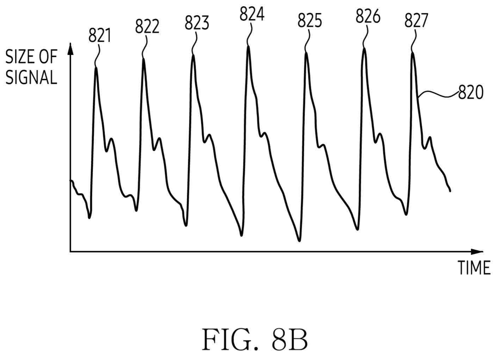

FIG. 8A exemplifies a location at which a wearable device is worn, according to an embodiment. FIG. 8B exemplifies biometric data obtained by a wearable device according to an embodiment.

In an embodiment, a processor 120 may obtain an input requesting for obtaining biometric information. Herein, the input requesting for obtaining biometric information may be a trigger instructing to obtain biometric information. The input requesting for obtaining biometric information may be generated from the processor 120 based on satisfaction of a designated condition. For example, in a case that obtaining of the biometric information is required periodically or aperiodically, the processor 120 may generate the input requesting for obtaining biometric information periodically or aperiodically. The input requesting for obtaining biometric information may be obtained from a user. For example, the processor 120 may obtain a user input requesting for obtaining biometric information obtained based on an input module (e.g., an input module 1450 of FIG. 14) provided in a wearable device 101. The input requesting for obtaining biometric information may be obtained from an electronic device 102.

In an embodiment, the processor 120 may identify whether a wearing state is a designated wearing state for obtaining biometric data. In an embodiment, the processor 120 may identify whether the wearing state is the designated wearing state for obtaining biometric data in response to obtaining the input requesting for obtaining biometric information. In an embodiment, the processor 120 may identify whether a wearing state is a wearing state corresponding to a type of biometric information requested for obtaining among a plurality of wearing states. Herein, a wearing state corresponding according to the type of the biometric information requested for obtaining may be different. The number of wearing state corresponding to a heart rate may be greater than the number of wearing state corresponding to saturation of percutaneous oxygen. For example, the heart rate may be obtained in all of a tight state, a normal state, or a loose state. For example, the saturation of percutaneous oxygen may be obtained in the tight state or the normal state.

For example, in a case that the biometric information requested for obtaining is the saturation of percutaneous oxygen and the wearing state is the loose state, the processor 120 may identify that the wearing state is different from the designated wearing state. For example, in a case that the biometric information requested for obtaining is the saturation of percutaneous oxygen and the wearing state is the tight state or the normal state, the processor 120 may identify that the wearing state is the designated wearing state.

For example, referring to FIG. 8A, fingers 811, 813, 815, 817, and 819 of a hand 810 of the user may have different thicknesses (or circumferences). For example, a thickness (or a circumference) of the thumb 811 may decrease from the thumb 811 to the little finger 819. For example, in a case that the wearable device 101 is worn on the ring finger 817 or the little finger 819, the processor 120 may identify that a wearing state of the wearable device 101 is the loose state. For example, in a case that the wearable device 101 is worn on the middle finger 815, the processor 120 may identify that the wearing state of the wearable device 101 is the normal state. For example, in a case that the wearable device 101 is worn on the thumb 811 or the index finger 813, the processor 120 may identify that the wearing state of the wearable device 101 is the tight state.

In an embodiment, the processor 120 may output a notification to guide a change in the wearing state in response to identifying that the wearing state is a different wearing state from the designated wearing state. The processor 120 may enable the electronic device 102 to output the notification to guide the change in the wearing state. For example, the processor 120 may transmit data indicating the identified wearing state to the electronic device 102 based on a communication connection with the electronic device 102. The electronic device 102 may output the notification to guide the change in the wearing state based on the data indicating the identified wearing state received from the wearable device 101. However, it is not limited thereto. The processor 120 may output the notification to guide the change in the wearing state through the wearable device 101. For example, the processor 120 may output the notification to guide the change in the wearing state through a component (e.g., a sound output module 1455, a display module 1460, and/or a haptic module 1479 of FIG. 14), which may interact with the user, included in the wearable device 101.

In an embodiment, the processor 120 may obtain biometric data through a biometric sensor 171 in response to identifying that the wearing state is the designated wearing state. For example, the processor 120 may obtain biometric data by obtaining reflected light of light emitted through at least one light emitting unit 22, 24, and/or 26 through at least one light receiving unit 23, 25, and/or 27. Herein, the biometric data may include pulse wave information according to a change in a blood flow rate of a body 100 by which the wearable device 101 is worn. The biometric data may include information related to a waveform of a pulse wave in which a blood flow rate flowing through a blood vessel changes over time as a heart repeatedly contracts and relaxes. For example, the processor 120 may obtain biometric data including a waveform 820 of a pulse wave exemplified in FIG. 8B through the biometric sensor 171.

In an embodiment, the processor 120 may obtain biometric information based on the obtained biometric data. In an embodiment, the processor 120 may obtain biometric information based on a waveform of a pulse wave indicated by the obtained biometric data. For example, the processor 120 may obtain heart rate information (or a heart rate) based on the number of peak values of the waveform of the pulse wave. For example, the processor 120 may obtain saturation of percutaneous oxygen information based on a ratio between intensity of a pulse wave indicated by reflected light of infrared light and intensity of a pulse wave indicated by reflected light of red light.

In an embodiment, the processor 120 may select a coefficient corresponding to the identified wearing state among a plurality of coefficients. For example, the processor 120 may select a coefficient corresponding to a wearing state for correcting a value indicated by the biometric information. Herein, a plurality of coefficients for a plurality of wearing states may be set in advance for at least one of a heart rate, saturation of percutaneous oxygen, arterial stiffness, blood pressure, an arterial age, stress, blood sugar, or IHRN. For example, for the heart rate, coefficients corresponding to the tight state, the normal state, or the loose state may be set in advance. Herein, the coefficient may be a value for compensating for the pulse wave not being accurately measured from the tight state to the loose state. For example, referring to FIG. 8B, the processor 120 may identify all peaks 821, 822, 823, 824, 825, 826, and 827, indicated by the waveform 820 of the pulse wave through biometric data obtained in the tight state or the normal state. For example, referring to FIG. 8B, at least a portion of a waveform of the waveform 820 of the pulse wave may not be lost in the biometric data obtained through the biometric sensor 171 in the tight state or the normal state. The processor 120 may identify only a portion of all the peaks 821, 822, 823, 824, 825, 826, and 827 indicated by the waveform 820 of the pulse wave through the biometric data obtained in the loose state. For example, at least some waveform of the waveform 820 of the pulse wave may be lost in the biometric data obtained through the biometric sensor 171 in the loose state. Accordingly, some of the peaks 821, 822, 823, 824, 825, 826, and 827 may not be identified in the loose state.

In an embodiment, the processor 120 may adjust the obtained biometric information, based on the selected coefficient. The processor 120 may adjust biometric information for at least one of a heart rate, saturation of percutaneous oxygen, arterial stiffness, blood pressure, an arterial age, stress, blood sugar, or IHRN based on the selected coefficient. For example, the processor 120 may increase the heart rate based on a coefficient corresponding to the loose state. For example, the processor 120 may adjust the heart rate by multiplying a heart rate obtained in the loose state by the coefficient corresponding to the loose state.

Hereinafter, an operation obtaining information on a finger of the user on which the wearable device 101 is worn will be described.

In an embodiment, the processor 120 may identify the wearing state of the wearable device 101. For example, the processor 120 may identify which finger of the user the wearable device 101 is worn on. For example, when the wearable device 101 is worn in a wearing release state, the processor 120 may identify which finger of the user the wearable device 101 is worn on. For example, based on a wearing state identified when the wearable device 101 is worn in the wearing release state, the processor 120 may identify which finger of the user the wearable device 101 is worn on.

In an embodiment, the processor 120 may identify which finger of the user the wearable device 101 is worn on, based on information related to the body 100 of the user. For example, the processor 120 may identify which finger of the user the wearable device 101 is worn on, based on information on a thickness (or a circumference) of a finger of the user. For example, the processor 120 may identify which finger of the user the wearable device 101 is worn on by comparing a finger thickness (or circumference) indicated by a wearing state with information on a finger thickness (or circumference) of the user. For example, in a case that a circumference of the index finger of the user is approximately 57 millimeters, a circumference of the middle finger is approximately 56 millimeters, and a circumference of the ring finger is approximately 55 millimeters, the processor 120 may identify that the wearable device 101 is worn on a finger corresponding to a change in a temperature value indicated by temperature data. For example, when the wearable device 101 is worn on a finger having a circumference of approximately 57 millimeters, the processor 120 may determine the wearing state of the wearable device 101 as the tight state. When the wearable device 101 is worn on a finger having a circumference of approximately 56 millimeters, the processor 120 may determine the wearing state of the wearable device 101 as the normal state. When the wearable device 101 is worn on a finger having a circumference of approximately 55 millimeters, the processor 120 may determine the wearing state of the wearable device 101 as the loose state.

According to an embodiment, in a case of detecting wearing of the wearable device 101 after guiding the user to wear it on a designated finger, the processor 120 may obtain information on a thickness (or a circumference) of the designated finger of the user. In addition, in a case of detecting wearing of the wearable device 101 after guiding the user to wear it on the designated finger, the processor 120 may obtain information on the thickness (or the circumference) of the designated finger of the user and/or a change in the thickness (or the circumference) of the finger during the user's sleep.

Hereinafter, an operation providing a notification based on a finger of the user on which the wearable device 101 is worn will be described.

In an embodiment, the processor 120 may obtain a user input based on an interaction between the wearable device 101 and the user. For example, in a case of pointing to a point through the body 100 or moving to a designated path in a state in which the wearable device 101 is worn by the body 100 of the user, the processor 120 may obtain an operation using the body 100 as a user input. However, in a case that the wearable device 101 is not worn at a suitable location (e.g., the index finger) for the body 100, the user input may not be properly obtained. Accordingly, it is required to guide the wearable device 101 to be worn on the suitable finger of the user such that the user may perform a gesture operation smoothly. An operation for this may be described below.

In an embodiment, the processor 120 may identify the wearing state of the wearable device 101. For example, the processor 120 may identify which finger of the user the wearable device 101 is worn on. For example, when the wearable device 101 is worn in the wearing release state, the processor 120 may identify which finger of the user the wearable device 101 is worn on. For example, based on a wearing state identified when the wearable device 101 is worn in the wearing release state, the processor 120 may identify which finger of the user the wearable device 101 is worn on.

In an embodiment, the processor 120 may determine whether the wearable device 101 is worn on a designated finger of the user. Herein, the designated finger may be a designated finger (e.g., the index finger) for a gesture input.

In an embodiment, the processor 120 may output a notification to guide a change in the wearing state in response to identifying that the wearable device 101 is worn on a finger other than the designated finger of the user. The processor 120 may enable the electronic device 102 to output the notification to guide the change in the wearing state. For example, the processor 120 may transmit data indicating the identified wearing state to the electronic device 102 based on the communication connection with the electronic device 102. The electronic device 102 may output the notification to guide the change in the wearing state based on the data indicating the identified wearing state received from the wearable device 101. However, it is not limited thereto. The processor 120 may output the notification to guide the change in the wearing state through the wearable device 101. For example, the processor 120 may output the notification to guide the change in the wearing state through the component (e.g., the sound output module 1455, the display module 1460, and/or the haptic module 1479 of FIG. 14), which may interact with the user, included in the wearable device 101.

In an embodiment, the processor 120 may obtain movement data indicating movement of the wearable device 101 through a motion sensor 173 in response to identifying that the wearable device 101 is worn on the designated finger of the user. For example, the processor 120 may obtain movement data indicating three-axis movement (e.g., a change in acceleration in a space or a change in angular velocity in a space). For example, the processor 120 may obtain movement data indicating six-axis movement (e.g., a change in acceleration in a space and a change in angular velocity in a space).

In an embodiment, the processor 120 may identify a gesture corresponding to a movement path of the wearable device 101 indicated by the movement data. In an embodiment, the processor 120 may obtain the gesture corresponding to the movement path of the wearable device 101 indicated by the movement data as a user input.

Hereinafter, an operation in which the wearable device 101 provides a notification based on a location of the worn hand will be described.

In an embodiment, the processor 120 may obtain a movement trajectory of the body 100 in a state in which the wearable device 101 is worn by the body 100 of the user. The movement trajectory of the body 100 may be fed back to the user. For example, in order to provide the user with information on whether a golf swing, a tennis stroke, and/or a baseball pitching motion has performed properly, the processor 120 may feed back the movement trajectory of the body 100 to the user. To this end, it is required to wear the wearable device 101 on a finger of a main hand designated by the user. Accordingly, it is required to guide the wearable device 101 to be worn on a suitable hand of the user such that the movement trajectory may be properly collected during exercise of the user. An operation for this may be described below.

In an embodiment, the processor 120 may identify a wearing state of a wearable device 101.

In an embodiment, the processor 120 may obtain movement data indicating movement of the wearable device 101 through the motion sensor 173 in response to identifying that the wearable device 101 is worn by the user. For example, the processor 120 may obtain movement data indicating three-axis movement (e.g., a change in acceleration in a space or a change in angular velocity in a space). For example, the processor 120 may obtain movement data indicating six-axis movement (e.g., a change in acceleration in a space and a change in angular velocity in a space).

In an embodiment, the processor 120 may obtain a tracking input for tracking a movement path (or a movement path of the hand (or an arm) of the user) of the wearable device 101. In an embodiment, the processor 120 may obtain movement data indicating movement of the wearable device 101 through the motion sensor 173 in response to the tracking input.

In an embodiment, the processor 120 may identify a movement path of the wearable device 101 indicated by the movement data.

In an embodiment, the processor 120 may identify a location of the hand on which the wearable device 101 is worn based on the movement path of the wearable device 101 indicated by the movement data. For example, the processor 120 may identify a hand movable to the movement path of the wearable device 101 among both hands as a hand on which the wearable device 101 is worn. For example, in a case that at least some movement path of the movement path of the wearable device 101 is outside an operation range of any arm among a left arm or a right arm, the processor 120 may identify that the wearable device 101 is worn on a hand of another arm. However, it is not limited thereto.

In an embodiment, the processor 120 may determine whether the wearable device 101 is worn on a designated hand of the user. Herein, the designated hand may be a main use hand of the user. The main use hand may be a hand designated for exercise (e.g., golf, tennis, and baseball) of the user.

In an embodiment, the processor 120 may output a notification to guide a change in the wearing state in response to identifying that the wearable device 101 is worn on a hand other than the designated hand of the user. The processor 120 may enable the electronic device 102 to output the notification to guide the change in the wearing state. For example, the processor 120 may transmit data indicating the identified wearing state to the electronic device 102 based on the communication connection with the electronic device 102. The electronic device 102 may output the notification to guide the change in the wearing state based on the data indicating the identified wearing state received from the wearable device 101. However, it is not limited thereto. The processor 120 may output the notification to guide the change in the wearing state through the wearable device 101. For example, the processor 120 may output the notification to guide the change in the wearing state through the component (e.g., the sound output module 1455, the display module 1460, and/or the haptic module 1479 of FIG. 14), which may interact with the user, included in the wearable device 101.

In an embodiment, the processor 120 may provide the user with the movement data in response to identifying that the wearable device 101 is worn on the designated hand of the user. For example, the processor 120 may enable the electronic device 102 to display a movement trajectory according to the movement data. For example, the processor 120 may transmit the movement data to the electronic device 102 based on the communication connection with the electronic device 102. The electronic device 102 may display the movement trajectory based on the movement data received from the wearable device 101. However, it is not limited thereto.

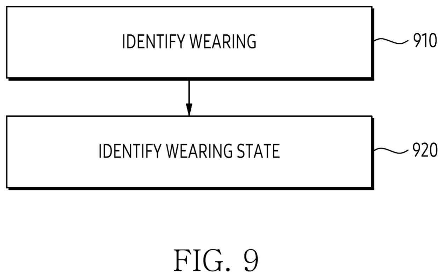

FIG. 9 is a flowchart indicating an operation of a wearable device according to an embodiment.

In the following embodiment, each of operations may be sequentially performed, but is not necessarily performed sequentially. For example, an order of each of the operations may also be changed, and at least two operations may also be performed in parallel.

FIG. 9 may be described with reference to FIGS. 1 to 6B.

Referring to FIG. 9, in operation 910, a processor 120 may identify wearing. In an embodiment, the processor 120 may identify that a wearable device 101 is worn by a user based on a temperature sensor 170 and/or a proximity sensor 172. The processor 120 may identify that the wearable device 101 is worn by the user while a state of the wearable device 101 is a wearing release 410.

In an embodiment, the processor 120 may identify that the wearable device 101 is worn by the user based on the temperature sensor 170. In an embodiment, the processor 120 may identify that the wearable device 101 is worn by the user based on a temperature value obtained through the temperature sensor 170. For example, the processor 120 may identify that the wearable device 101 is worn by the user based on whether the temperature value changes more than or equal to a reference temperature value. For example, the processor 120 may identify that the wearable device 101 is worn by the user based on whether the temperature value changes more than or equal to the reference temperature value within a designated time period.

In an embodiment, the processor 120 may identify that the wearable device 101 is worn by the user based on the proximity sensor 172. In an embodiment, the processor 120 may identify that the wearable device 101 is worn by the user based on an intensity value indicating intensity of light obtained through the proximity sensor 172. For example, the processor 120 may identify that the wearable device 101 is worn by the user based on whether the intensity value is more than or equal to a reference intensity value. For example, in a case that the intensity value is more than or equal to the reference intensity value, the processor 120 may identify that the wearable device 101 is worn by the user.

In an embodiment, the processor 120 may identify that the wearable device 101 is worn by the user based on the temperature sensor 170 and the proximity sensor 172. The processor 120 may identify a first state of the wearable device 101 based on the temperature sensor 170. The processor 120 may identify a second state of the wearable device 101 based on the proximity sensor 172. In a case that the first state indicates that the wearable device 101 is worn by the user, and the second state indicates that the wearable device 101 is worn by the user, the processor 120 may identify that the wearable device 101 is worn by the user. In a case that at least one state among the first state or the second state indicates that the wearable device 101 is not worn by the user, the processor 120 may identify that the wearable device 101 is not worn by the user.

In operation 920, the processor 120 may identify a wearing state. In an embodiment, the processor 120 may identify a wearing state of the wearable device 101. In response to identifying that the wearable device 101 is worn by the user, the processor 120 may identify the wearing state of the wearable device 101. Herein, the wearing state may be configured with a plurality of wearing states. The plurality of wearing states may indicate different degrees of loosening (or degrees of tightness) between the wearable device 101 and a body 100. The processor 120 may identify the wearing state of the wearable device 101 while a state of the wearable device 101 is wearing 420.

For example, a plurality of wearing states 601, 603, and 605 may correspond to a plurality of finger circumferences. For example, the plurality of wearing states 601, 603, and 605 may correspond to a state of being worn by each of fingers having a finger circumference of a designated range. For example, the plurality of wearing states 601, 603, and 605 may be set to have 1 millimeter interval in the designated range. However, it is not limited thereto.

For example, the plurality of wearing states 601, 603, and 605 may be divided into N wearing states. For example, the plurality of wearing states 601, 603, and 605 may include N-2 wearing states between the tight state 605 and the loose state 601. For example, in a case that the N is 3, the plurality of wearing states 601, 603, and 605 may be divided into three wearing states.

The processor 120 may identify the wearing state of the wearable device 101 based on the temperature sensor 170. The processor 120 may identify one wearing state corresponding to temperature data indicating a temperature value measured through the temperature sensor 170 among the plurality of wearing states. For example, the processor 120 may identify one wearing state corresponding to a change in a temperature value indicated by temperature data among the plurality of wearing states 601, 603, and 605. For example, the processor 120 may identify one wearing state corresponding to a change in a temperature value indicated by temperature data obtained for a designated time period. For example, the processor 120 may identify one wearing state corresponding to variance, deviation, and/or standard deviation of the temperature value indicated by the temperature data obtained for the designated time period. For example, in a case that a range for each of the plurality of wearing states is set, the processor 120 may identify a wearing state of a range to which a value (e.g., variance, deviation, and/or standard deviation) indicating a change in a temperature value belongs as a wearing state of the wearable device 101.

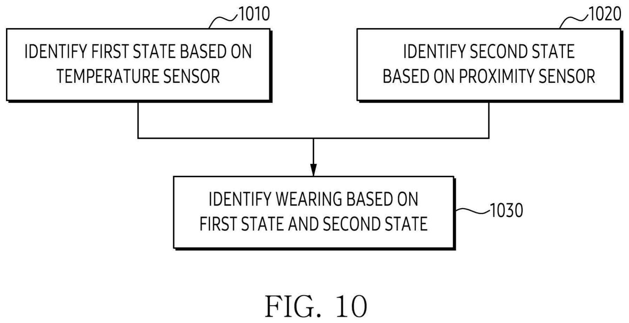

FIG. 10 is a flowchart indicating an operation of a wearable device according to an embodiment.

In the following embodiment, each of operations may be sequentially performed, but is not necessarily performed sequentially. For example, an order of each of the operations may also be changed, and at least two operations may also be performed in parallel.

FIG. 10 may be described with reference to FIGS. 1 to 6B.

Referring to FIG. 10, in operation 1010, a processor 120 may identify a first state based on a temperature sensor 170. The processor 120 may identify the first state of a wearable device 101 based on the temperature sensor 170. Herein, the first state of the wearable device 101 may indicate one of a wearing release 410 of the wearable device 101 or wearing 420 of the wearable device 101.

In an embodiment, the processor 120 may identify the first state of the wearable device 101 based on the temperature value obtained through the temperature sensor 170. For example, the processor 120 may identify the first state of the wearable device 101 based on whether the temperature value changes more than or equal to a reference temperature value (e.g., 4 degrees). For example, the processor 120 may identify the first state of the wearable device 101 based on whether the temperature value changes more than or equal to the reference temperature value within a designated time period (e.g., 10 seconds).

In operation 1020, the processor 120 may identify a second state based on a proximity sensor 172. The processor 120 may identify the second state of the wearable device 101 based on the proximity sensor 172. Herein, the second state of the wearable device 101 may indicate one of the wearing release 410 of the wearable device 101 or the wearing 420 of the wearable device 101.

In an embodiment, the processor 120 may identify the second state of the wearable device 101 based on an intensity value indicating intensity of light obtained through the proximity sensor 172. For example, the processor 120 may identify the second state of the wearable device 101 based on whether the intensity value is more than or equal to a reference intensity value.

In operation 1030, the processor 120 may identify wearing based on the first state and the second state.

In a case that the first state indicates that the wearable device 101 is worn by the user, and the second state indicates that the wearable device 101 is worn by the user, the processor 120 may identify that the wearable device 101 is worn by the user. In a case that at least one state among the first state or the second state indicates that the wearable device 101 is not worn by the user, the processor 120 may identify that the wearable device 101 is not worn by the user.

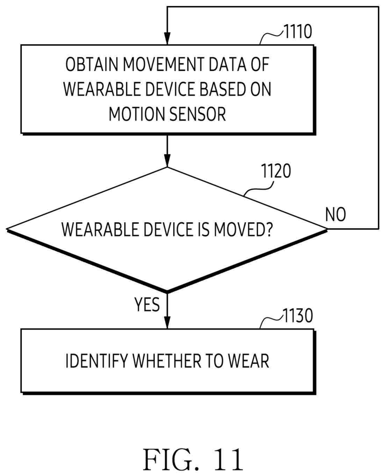

FIG. 11 is a flowchart indicating an operation of a wearable device according to an embodiment.

In the following embodiment, each of operations may be sequentially performed, but is not necessarily performed sequentially. For example, an order of each of the operations may also be changed, and at least two operations may also be performed in parallel.

FIG. 11 may be described with reference to FIGS. 1 to 6B.

Referring to FIG. 11, in operation 1110, a processor 120 may obtain movement data of a wearable device 101 based on a motion sensor 173. In an embodiment, the processor 120 may obtain movement data indicating movement of the wearable device 101 through the motion sensor 173 while a state of the wearable device 101 is a wearing release 410. In an embodiment, the processor 120 may obtain movement data indicating the movement of the wearable device 101 through the motion sensor 173 while operating in a low-power mode (or a sleep mode). For example, the processor 120 may obtain movement data indicating three-axis movement (e.g., a change in acceleration in a space or a change in angular velocity in a space). For example, the processor 120 may obtain movement data indicating six-axis movement (e.g., a change in acceleration in a space and a change in angular velocity in a space).

In operation 1120, the processor 120 may determine whether the wearable device 101 is moved. The processor 120 may determine whether the wearable device 101 moves more than or equal to a designated distance (e.g., 50 cm) based on the movement data. In a case that the wearable device 101 moves more than or equal to the designated distance, the processor 120 may determine that the wearable device 101 has moved. In a case that the wearable device 101 moves less than the designated distance, the processor 120 may determine that the wearable device 101 has not moved.

In the operation 1120, when it is determined that the wearable device 101 has moved, the processor 120 may perform operation 1130. In the operation 1120, when it is determined that the wearable device 101 has not moved, the processor 120 may perform the operation 1110 again.

In the operation 1130, the processor 120 may identify whether to wear the wearable device. The processor 120 may determine whether the wearable device 101 is worn by the user based on the movement data indicating the movement of the wearable device 101. The processor 120 may identify whether the wearable device 101 is worn by the user in response to the movement data indicating the movement of the wearable device 101. For example, in a case that the wearable device 101 is identified as being moved more than or equal to the designated distance based on the movement data, the processor 120 may identify whether the wearable device 101 is worn by the user. For example, the processor 120 may identify that the wearable device 101 is worn by the user based on a temperature sensor 170 and/or a proximity sensor 172. The processor 120 may identify that the wearable device 101 is worn by the user while a state of the wearable device 101 is the wearing release 410.

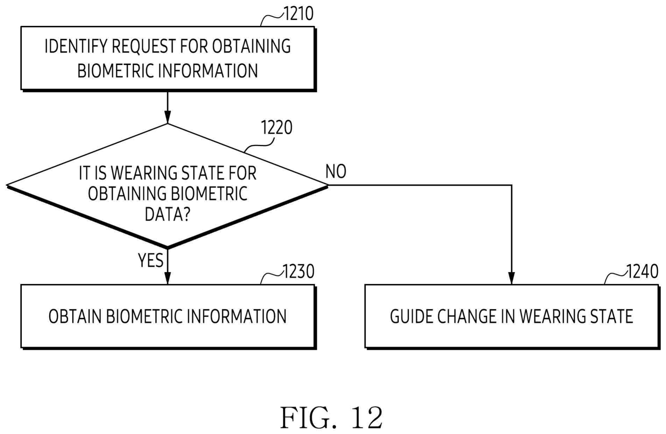

FIG. 12 is a flowchart indicating an operation of a wearable device according to an embodiment.

In the following embodiment, each of operations may be sequentially performed, but is not necessarily performed sequentially. For example, an order of each of the operations may also be changed, and at least two operations may also be performed in parallel.

FIG. 12 may be described with reference to FIGS. 1 to 6B.

Referring to FIG. 12, in operation 1210, a processor 120 may identify a request for obtaining biometric information. Herein, an input requesting for obtaining biometric information may be a trigger instruct to obtain biometric information. The input requesting for obtaining biometric information may be generated from the processor 120 based on satisfaction of a designated condition. For example, in a case that obtaining of the biometric information is required periodically or aperiodically, the processor 120 may generate the input requesting for obtaining biometric information periodically or aperiodically. The input requesting for obtaining biometric information may be obtained from a user. For example, the processor 120 may obtain a user input requesting for obtaining biometric information obtained based on an input module (e.g., an input module 1450 of FIG. 14) provided in a wearable device 101. The input requesting for obtaining biometric information may be obtained from an electronic device 102.

In operation 1220, the processor 120 may determine whether it is a wearing state for obtaining biometric data. In an embodiment, the processor 120 may identify whether the wearing state of the wearable device 101 is a designated wearing state for obtaining biometric data. In an embodiment, the processor 120 may identify whether the wearing state is the designated wearing state for obtaining biometric data in response to obtaining the input requesting for obtaining biometric information. In an embodiment, the processor 120 may identify whether a wearing state is a wearing state corresponding to a type of biometric information requested for obtaining among a plurality of wearing states. Herein, a wearing state corresponding according to the type of the biometric information requested for obtaining may be different. The number of wearing state corresponding to a heart rate may be greater than the number of wearing state corresponding to saturation of percutaneous oxygen. For example, the heart rate may be obtained in all of a tight state, a normal state, or a loose state. For example, the saturation of percutaneous oxygen may be obtained in the tight state or the normal state.

In the operation 1220, when it is determined that the wearing state of the wearable device 101 is the wearing state for obtaining the biometric data, the processor 120 may perform operation 1230. In the operation 1220, when it is determined that the wearing state of the wearable device 101 is not the wearing state for obtaining the biometric data, the processor 120 may perform operation 1240.

In the operation 1230, the processor 120 may obtain biometric information.

In an embodiment, the processor 120 may obtain biometric data through a biometric sensor 171 in response to identifying that the wearing state is the designated wearing state. Herein, the biometric data may include pulse wave information according to a change in a blood flow rate of a body 100 by which the wearable device 101 is worn.

In an embodiment, the processor 120 may obtain biometric information based on the obtained biometric data. In an embodiment, the processor 120 may obtain biometric information based on a waveform of a pulse wave indicated by the obtained biometric data. For example, the processor 120 may obtain heart rate information (or a heart rate) based on the number of peak values of the waveform of the pulse wave. For example, the processor 120 may obtain saturation of percutaneous oxygen information based on a ratio between intensity of a pulse wave indicated by reflected light of infrared light and intensity of a pulse wave indicated by reflected light of red light.

In the operation 1240, the processor 120 may guide a change in the wearing state. In an embodiment, the processor 120 may output a notification to guide a change in the wearing state in response to identifying that the wearing state is different from the wearing state for obtaining biometric data. The processor 120 may enable the electronic device 102 to output the notification to guide the change in the wearing state. For example, the processor 120 may transmit data indicating the identified wearing state to the electronic device 102 based on a communication connection with the electronic device 102. The electronic device 102 may output the notification to guide the change in the wearing state based on the data indicating the identified wearing state received from the wearable device 101. However, it is not limited thereto.

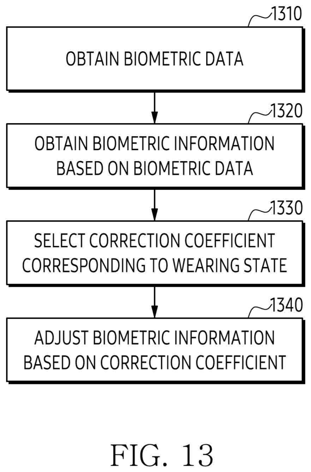

FIG. 13 is a flowchart indicating an operation of a wearable device according to an embodiment.

In the following embodiment, each of operations may be sequentially performed, but is not necessarily performed sequentially. For example, an order of each of the operations may also be changed, and at least two operations may also be performed in parallel.

FIG. 13 may be described with reference to FIGS. 1 to 6B.

Referring to FIG. 13, in operation 1310, a processor 120 may obtain biometric data. In an embodiment, the processor 120 may obtain biometric data through a biometric sensor 171. In an embodiment, the processor 120 may obtain biometric data through the biometric sensor 171 in response to identifying that a wearing state is a designated wearing state. Herein, the biometric data may include pulse wave information according to a change in a blood flow rate of a body 100 by which a wearable device 101 is worn.

In operation 1320, the processor 120 may obtain biometric information based on the biometric data. In an embodiment, the processor 120 may obtain biometric information based on a waveform of a pulse wave indicated by the obtained biometric data. For example, the processor 120 may obtain heart rate information (or a heart rate) based on the number of peak values of the waveform of the pulse wave. For example, the processor 120 may obtain saturation of percutaneous oxygen information based on a ratio between intensity of a pulse wave indicated by reflected light of infrared light and intensity of a pulse wave indicated by reflected light of red light.

In operation 1330, the processor 120 may select a correction coefficient corresponding to the wearing state. In an embodiment, the processor 120 may select a correction coefficient corresponding to the identified wearing state among a plurality of correction coefficients. For example, the processor 120 may select a coefficient corresponding to a wearing state for correcting a value indicated by the biometric information. Herein, a plurality of correction coefficients for a plurality of wearing states may be set in advance for at least one of a heart rate, saturation of percutaneous oxygen, arterial stiffness, blood pressure, an arterial age, stress, blood sugar, or IHRN. For example, for the heart rate, correction coefficients corresponding to a tight state, a normal state, or a loose state may be set in advance. Herein, the correction coefficient may be a value for compensating for the pulse wave not being accurately measured from the tight state to the loose state.

In operation 1340, the processor 120 may adjust the biometric information based on the correction coefficient. The processor 120 may adjust biometric information for at least one of a heart rate, saturation of percutaneous oxygen, arterial stiffness, blood pressure, an arterial age, stress, blood sugar, or IHRN based on the selected correction coefficient. For example, the processor 120 may increase the heart rate based on a coefficient corresponding to the loose state. For example, the processor 120 may adjust the heart rate by multiplying a heart rate obtained in the loose state by the coefficient corresponding to the loose state.

FIG. 14 is a block diagram illustrating an electronic device 1401 in a network environment 1400 according to various embodiments.

Referring to FIG. 14, the electronic device 1401 in the network environment 1400 may communicate with an electronic device 1402 via a first network 1498 (e.g., a short-range wireless communication network), or at least one of an electronic device 1404 or a server 1408 via a second network 1499 (e.g., a long-range wireless communication network). According to an embodiment, the electronic device 1401 may communicate with the electronic device 1404 via the server 1408. According to an embodiment, the electronic device 1401 may include a processor 1420, memory 1430, an input module 1450, a sound output module 1455, a display module 1460, an audio module 1470, a sensor module 1476, an interface 1477, a connecting terminal 1478, a haptic module 1479, a camera module 1480, a power management module 1488, a battery 1489, a communication module 1490, a subscriber identification module(SIM) 1496, or an antenna module 1497. In some embodiments, at least one of the components (e.g., the connecting terminal 1478) may be omitted from the electronic device 1401, or one or more other components may be added in the electronic device 1401. In some embodiments, some of the components (e.g., the sensor module 1476, the camera module 1480, or the antenna module 1497) may be implemented as a single component (e.g., the display module 1460).

The processor 1420 may execute, for example, software (e.g., a program 1440) to control at least one other component (e.g., a hardware or software component) of the electronic device 1401 coupled with the processor 1420, and may perform various data processing or computation. According to an embodiment, as at least part of the data processing or computation, the processor 1420 may store a command or data received from another component (e.g., the sensor module 1476 or the communication module 1490) in volatile memory 1432, process the command or the data stored in the volatile memory 1432, and store resulting data in non-volatile memory 1434. According to an embodiment, the processor 1420 may include a main processor 1421 (e.g., a central processing unit (CPU) or an application processor (AP)), or an auxiliary processor 1423 (e.g., a graphics processing unit (GPU), a neural processing unit (NPU), an image signal processor (ISP), a sensor hub processor, or a communication processor (CP)) that is operable independently from, or in conjunction with, the main processor 1421. For example, when the electronic device 1401 includes the main processor 1421 and the auxiliary processor 1423, the auxiliary processor 1423 may be adapted to consume less power than the main processor 1421, or to be specific to a specified function. The auxiliary processor 1423 may be implemented as separate from, or as part of the main processor 1421.

The auxiliary processor 1423 may control at least some of functions or states related to at least one component (e.g., the display module 1460, the sensor module 1476, or the communication module 1490) among the components of the electronic device 1401, instead of the main processor 1421 while the main processor 1421 is in an inactive (e.g., sleep) state, or together with the main processor 1421 while the main processor 1421 is in an active state (e.g., executing an application). According to an embodiment, the auxiliary processor 1423 (e.g., an image signal processor or a communication processor) may be implemented as part of another component (e.g., the camera module 1480 or the communication module 1490) functionally related to the auxiliary processor 1423. According to an embodiment, the auxiliary processor 1423 (e.g., the neural processing unit) may include a hardware structure specified for artificial intelligence model processing. An artificial intelligence model may be generated by machine learning. Such learning may be performed, e.g., by the electronic device 1401 where the artificial intelligence is performed or via a separate server (e.g., the server 1408). Learning algorithms may include, but are not limited to, e.g., supervised learning, unsupervised learning, semi-supervised learning, or reinforcement learning. The artificial intelligence model may include a plurality of artificial neural network layers. The artificial neural network may be a deep neural network (DNN), a convolutional neural network (CNN), a recurrent neural network (RNN), a restricted Boltzmann machine (RBM), a deep belief network (DBN), a bidirectional recurrent deep neural network (BRDNN), deep Q-network or a combination of two or more thereof but is not limited thereto. The artificial intelligence model may, additionally or alternatively, include a software structure other than the hardware structure.

The memory 1430 may store various data used by at least one component (e.g., the processor 1420 or the sensor module 1476) of the electronic device 1401. The various data may include, for example, software (e.g., the program 1440) and input data or output data for a command related thereto. The memory 1430 may include the volatile memory 1432 or the non-volatile memory 1434.

The program 1440 may be stored in the memory 1430 as software, and may include, for example, an operating system (OS) 1442, middleware 1444, or an application 1446.

The input module 1450 may receive a command or data to be used by another component (e.g., the processor 1420) of the electronic device 1401, from the outside (e.g., a user) of the electronic device 1401. The input module 1450 may include, for example, a microphone, a mouse, a keyboard, a key (e.g., a button), or a digital pen (e.g., a stylus pen).

The sound output module 1455 may output sound signals to the outside of the electronic device 1401. The sound output module 1455 may include, for example, a speaker or a receiver. The speaker may be used for general purposes, such as playing multimedia or playing record. The receiver may be used for receiving incoming calls. According to an embodiment, the receiver may be implemented as separate from, or as part of the speaker.

The display module 1460 may visually provide information to the outside (e.g., a user) of the electronic device 1401. The display module 1460 may include, for example, a display, a hologram device, or a projector and control circuitry to control a corresponding one of the display, hologram device, and projector. According to an embodiment, the display module 1460 may include a touch sensor adapted to detect a touch, or a pressure sensor adapted to measure the intensity of force incurred by the touch.

The audio module 1470 may convert a sound into an electrical signal and vice versa. According to an embodiment, the audio module 1470 may obtain the sound via the input module 1450, or output the sound via the sound output module 1455 or a headphone of an external electronic device (e.g., an electronic device 1402) directly (e.g., wiredly) or wirelessly coupled with the electronic device 1401.

The sensor module 1476 may detect an operational state (e.g., power or temperature) of the electronic device 1401 or an environmental state (e.g., a state of a user) external to the electronic device 1401, and then generate an electrical signal or data value corresponding to the detected state. According to an embodiment, the sensor module 1476 may include, for example, a gesture sensor, a gyro sensor, an atmospheric pressure sensor, a magnetic sensor, an acceleration sensor, a grip sensor, a proximity sensor, a color sensor, an infrared (IR) sensor, a biometric sensor, a temperature sensor, a humidity sensor, or an illuminance sensor.

The interface 1477 may support one or more specified protocols to be used for the electronic device 1401 to be coupled with the external electronic device (e.g., the electronic device 1402) directly (e.g., wiredly) or wirelessly. According to an embodiment, the interface 1477 may include, for example, a high definition multimedia interface (HDMI), a universal serial bus (USB) interface, a secure digital (SD) card interface, or an audio interface.

A connecting terminal 1478 may include a connector via which the electronic device 1401 may be physically connected with the external electronic device (e.g., the electronic device 1402). According to an embodiment, the connecting terminal 1478 may include, for example, an HDMI connector, a USB connector, an SD card connector, or an audio connector (e.g., a headphone connector).

The haptic module 1479 may convert an electrical signal into a mechanical stimulus (e.g., a vibration or a movement) or electrical stimulus which may be recognized by a user via his tactile sensation or kinesthetic sensation. According to an embodiment, the haptic module 1479 may include, for example, a motor, a piezoelectric element, or an electric stimulator.

The camera module 1480 may capture a still image or moving images. According to an embodiment, the camera module 1480 may include one or more lenses, image sensors, image signal processors, or flashes.

The power management module 1488 may manage power supplied to the electronic device 1401. According to an embodiment, the power management module 1488 may be implemented as at least part of, for example, a power management integrated circuit (PMIC).

The battery 1489 may supply power to at least one component of the electronic device 1401. According to an embodiment, the battery 1489 may include, for example, a primary cell which is not rechargeable, a secondary cell which is rechargeable, or a fuel cell.

The communication module 1490 may support establishing a direct (e.g., wired) communication channel or a wireless communication channel between the electronic device 1401 and the external electronic device (e.g., the electronic device 1402, the electronic device 1404, or the server 1408) and performing communication via the established communication channel. The communication module 1490 may include one or more communication processors that are operable independently from the processor 1420 (e.g., the application processor (AP)) and supports a direct (e.g., wired) communication or a wireless communication. According to an embodiment, the communication module 1490 may include a wireless communication module 1492 (e.g., a cellular communication module, a short-range wireless communication module, or a global navigation satellite system (GNSS) communication module) or a wired communication module 1494 (e.g., a local area network (LAN) communication module or a power line communication (PLC) module). A corresponding one of these communication modules may communicate with the external electronic device via the first network 1498 (e.g., a short-range communication network, such as Bluetooth^{™}, wireless-fidelity (Wi-Fi) direct, or infrared data association (IrDA)) or the second network 1499 (e.g., a long-range communication network, such as a legacy cellular network, a 5G network, a next-generation communication network, the Internet, or a computer network (e.g., LAN or wide area network (WAN)). These various types of communication modules may be implemented as a single component (e.g., a single chip), or may be implemented as multi components (e.g., multi chips) separate from each other. The wireless communication module 1492 may identify and authenticate the electronic device 1401 in a communication network, such as the first network 1498 or the second network 1499, using subscriber information (e.g., international mobile subscriber identity (IMSI)) stored in the subscriber identification module 1496.

The wireless communication module 1492 may support a 5G network, after a 4G network, and next-generation communication technology, e.g., new radio (NR) access technology. The NR access technology may support enhanced mobile broadband (eMBB), massive machine type communications (mMTC), or ultra-reliable and low-latency communications (URLLC). The wireless communication module 1492 may support a high-frequency band (e.g., the mmWave band) to achieve, e.g., a high data transmission rate. The wireless communication module 1492 may support various technologies for securing performance on a high-frequency band, such as, e.g., beamforming, massive multiple-input and multiple-output (massive MIMO), full dimensional MIMO (FD-MIMO), array antenna, analog beam-forming, or large scale antenna. The wireless communication module 1492 may support various requirements specified in the electronic device 1401, an external electronic device (e.g., the electronic device 1404), or a network system (e.g., the second network 1499). According to an embodiment, the wireless communication module 1492 may support a peak data rate (e.g., 20Gbps or more) for implementing eMBB, loss coverage (e.g., 1464dB or less) for implementing mMTC, or U-plane latency (e.g., 0.5ms or less for each of downlink (DL) and uplink (UL), or a round trip of 14ms or less) for implementing URLLC.

The antenna module 1497 may transmit or receive a signal or power to or from the outside (e.g., the external electronic device) of the electronic device 1401. According to an embodiment, the antenna module 1497 may include an antenna including a radiating element composed of a conductive material or a conductive pattern formed in or on a substrate (e.g., a printed circuit board (PCB)). According to an embodiment, the antenna module 1497 may include a plurality of antennas (e.g., array antennas). In such a case, at least one antenna appropriate for a communication scheme used in the communication network, such as the first network 1498 or the second network 1499, may be selected, for example, by the communication module 1490 (e.g., the wireless communication module 1492) from the plurality of antennas. The signal or the power may then be transmitted or received between the communication module 1490 and the external electronic device via the selected at least one antenna. According to an embodiment, another component (e.g., a radio frequency integrated circuit (RFIC)) other than the radiating element may be additionally formed as part of the antenna module 1497.

According to various embodiments, the antenna module 1497 may form a mmWave antenna module. According to an embodiment, the mmWave antenna module may include a printed circuit board, an RFIC disposed on a first surface (e.g., the bottom surface) of the printed circuit board, or adjacent to the first surface and capable of supporting a designated high-frequency band (e.g., the mmWave band), and a plurality of antennas (e.g., array antennas) disposed on a second surface (e.g., the top or a side surface) of the printed circuit board, or adjacent to the second surface and capable of transmitting or receiving signals of the designated high-frequency band.

At least some of the above-described components may be coupled mutually and communicate signals (e.g., commands or data) therebetween via an inter-peripheral communication scheme (e.g., a bus, general purpose input and output (GPIO), serial peripheral interface (SPI), or mobile industry processor interface (MIPI)).

According to an embodiment, commands or data may be transmitted or received between the electronic device 1401 and the external electronic device 1404 via the server 1408 coupled with the second network 1499. Each of the electronic devices 1402 or 1404 may be a device of a same type as, or a different type, from the electronic device 1401. According to an embodiment, all or some of operations to be executed at the electronic device 1401 may be executed at one or more of the external electronic devices 1402, 1404, or 1408. For example, if the electronic device 1401 should perform a function or a service automatically, or in response to a request from a user or another device, the electronic device 1401, instead of, or in addition to, executing the function or the service, may request the one or more external electronic devices to perform at least part of the function or the service. The one or more external electronic devices receiving the request may perform the at least part of the function or the service requested, or an additional function or an additional service related to the request, and transfer an outcome of the performing to the electronic device 1401. The electronic device 1401 may provide the outcome, with or without further processing of the outcome, as at least part of a reply to the request. To that end, a cloud computing, distributed computing, mobile edge computing (MEC), or client-server computing technology may be used, for example. The electronic device 1401 may provide ultra low-latency services using, e.g., distributed computing or mobile edge computing. In another embodiment, the external electronic device 1404 may include an internet-of-things (IoT) device. The server 1408 may be an intelligent server using machine learning and/or a neural network. According to an embodiment, the external electronic device 1404 or the server 1408 may be included in the second network 1499. The electronic device 1401 may be applied to intelligent services (e.g., smart home, smart city, smart car, or healthcare) based on 5G communication technology or IoT-related technology.

A wearable device 101 as described above may include a temperature sensor 170 arranged to face skin of a user, when the wearable device 101 is worn by the user. The wearable device 101 may include at least one processor 120. The wearable device 101 may include memory 130 storing instructions. The instructions may be configured, when executed by the at least one processor 120, to cause the wearable device 101 to identify that the wearable device 101 is worn by the user. The instructions may be configured, when executed by the at least one processor 120, to cause the wearable device 101 to, in response to identifying that the wearable device 101 is worn by the user, identify one wearing state corresponding to temperature data among a plurality of wearing states 601, 603, and 605, the temperature data indicating a temperature value measured through the temperature sensor 170.

The wearable device 101 may further include a biometric sensor 171 obtaining biometric data related to the user. The instructions may be configured, when executed by the at least one processor 120, to cause the wearable device 101 to, in response to an input requesting for obtaining biometric information, identify whether the identified wearing state is a first wearing state for obtaining the biometric data. The instructions may be configured, when executed by the at least one processor 120, to cause the wearable device 101 to, in response to identifying that the identified wearing state is different from the first wearing state, output a notification to guide a change in the identified wearing state. The instructions may be configured, when executed by the at least one processor 120, to cause the wearable device 101 to, in response to identifying that the identified wearing state is the first wearing state, obtain biometric data through the biometric sensor 171. The instructions may be configured, when executed by the at least one processor 120, to cause the wearable device 101 to obtain the biometric information based on the obtained biometric data.

The wearable device 101 may further include a biometric sensor 171 obtaining biometric data related to the user. The instructions may be configured, when executed by the at least one processor 120, to cause the wearable device 101 to, in response to an input requesting for obtaining biometric information, obtain biometric data through the biometric sensor 171. The instructions may be configured, when executed by the at least one processor 120, to cause the wearable device 101 to obtain the biometric information based on the biometric data. The instructions may be configured, when executed by the at least one processor 120, to cause the wearable device 101 to select a coefficient corresponding to the identified wearing state among a plurality of coefficients. The instructions may be configured, when executed by the at least one processor 120, to cause the wearable device 101 to adjust the biometric information based on the selected coefficient.

The instructions may be configured, when executed by the at least one processor 120, to cause the wearable device 101 to identify that the wearable device 101 is worn by the user, based on that the temperature value changes more than or equal to a reference temperature value within a designated time period.

The wearable device 101 may further include a proximity sensor 172. The instructions may be configured, when executed by the at least one processor 120, to cause the wearable device 101 to identify a first state of the wearable device 101 based on a proximity sensing value obtained from the proximity sensor 172. The instructions may be configured, when executed by the at least one processor 120, to cause the wearable device 101 to identify a second state of the wearable device 101 based on that the temperature value changes more than or equal to the reference temperature value within the designated time period. The instructions may be configured, when executed by the at least one processor 120, to cause the wearable device 101 to identify that the wearable device 101 is worn by the user based on that both the first state and the second state indicate that the wearable device 101 is worn by the user.

The wearable device 101 may further include a motion sensor 173. The instructions may be configured, when executed by the at least one processor 120, to cause the wearable device 101 to obtain movement data indicating movement of the wearable device 101 through the motion sensor 173. The instructions may be configured, when executed by the at least one processor 120, to cause the wearable device 101 to, in response to that the movement data indicates that the wearable device 101 is moved, determine whether the wearable device 101 is being worn by the user.

The instructions may be configured, when executed by the at least one processor 120, to cause the wearable device 101 to, while the wearable device 101 is worn by the user, identify a change in the wearing state. The instructions may be configured, when executed by the at least one processor 120, to cause the wearable device 101 to identify that the changed wearing state is a second wearing state among the plurality of wearing states 601, 603, and 605. The instructions may be configured, when executed by the at least one processor 120, to cause the wearable device 101 to, based on identifying that the changed wearing state is the second wearing state, output a notification to guide a change in the wearing state.

The instructions may be configured, when executed by the at least one processor 120, to cause the wearable device 101 to, after identifying that the changed wearing state is the second wearing state, based on that the second wearing state is maintained more than or equal to a designated time period, output the notification.

The wearable device 101 may further include a pressure sensor 175 arranged to face the skin of the user. The instructions may be configured, when executed by the at least one processor 120, to cause the wearable device 101 to, based on identifying that the changed wearing state is the second wearing state, obtain a pressure signal through the pressure sensor 175. The instructions may be configured, when executed by the at least one processor 120, to cause the wearable device 101 to, based on that a pressure value indicated by the pressure signal exceeds a designated pressure value, output the notification.

The wearable device 101 may further include communication circuitry 190. The instructions may be configured, when executed by the at least one processor 120, to cause the wearable device 101 to establish a communication connection with an external electronic device 102 through the communication circuitry 190. The instructions may be configured, when executed by the at least one processor 120, to cause the wearable device 101 to, based on the communication connection, transmit data indicating the identified wearing state to the external electronic device 102 for the external electronic device 102 to output a notification to guide the identified wearing state to the user.

The instructions may be configured, when executed by the at least one processor 120, to cause the wearable device 101 to identify that the wearable device 101 is worn on a finger corresponding to the temperature data among fingers of the user.

The wearable device 101 may be configured to further include a motion sensor 173. The instructions may be configured, when executed by the at least one processor 120, to cause the wearable device 101 to, based on that the wearable device 101 is worn on a first finger among the fingers, obtain movement data indicating movement of the wearable device 101 through the motion sensor 173. The instructions may be configured, when executed by the at least one processor 120, to cause the wearable device 101 to, based on that the wearable device 101 is worn on the first finger among the fingers, identify a gesture corresponding to a movement path of the wearable device 101 indicated by the movement data. The instructions may be configured, when executed by the at least one processor 120, to cause the wearable device 101 to, based on identifying that the wearable device 101 is worn on a finger other than the first finger among the fingers, output a notification to guide a change in the identified wearing state.

The wearable device 101 may further include a motion sensor 173. The instructions may be configured, when executed by the at least one processor 120, to cause the wearable device 101 to obtain movement data indicating movement of the wearable device 101 through the motion sensor 173. The instructions may be configured, when executed by the at least one processor 120, to cause the wearable device 101 to, based on a movement path of the wearable device 101 indicated by the movement data, identify a hand where a finger on which the wearable device 101 is worn is located among both hands of the user.

The instructions may be configured, when executed by the at least one processor 120, to cause the wearable device 101 to obtain a tracking input for tracking a movement path. The instructions may be configured, when executed by the at least one processor 120, to cause the wearable device 101 to, in response to the tracking input, determine whether the identified hand is a designated hand. The instructions may be configured, when executed by the at least one processor 120, to cause the wearable device 101 to, in response to identifying that the identified hand is the designated hand, obtain movement data for tracking the movement path. The instructions may be configured, when executed by the at least one processor 120, to cause the wearable device 101 to, in response to identifying that the identified hand is different from the designated hand, output a notification to guide a change in the identified wearing state.

A method as described above may be performed by a wearable device 101 including a temperature sensor 170 arranged to face skin of a user, when the wearable device 101 is worn by the user. The method may include identifying that the wearable device 101 is worn by the user. The method may include, in response to identifying that the wearable device 101 is worn by the user, identifying one wearing state corresponding to temperature data among a plurality of wearing states 601, 603, and 605, the temperature data indicating a temperature value measured through the temperature sensor 170.

The method may include, in response to an input requesting for obtaining biometric information, identifying whether the identified wearing state is a first wearing state for obtaining the biometric data. The method may include, in response to identifying that the identified wearing state is different from the first wearing state, outputting a notification to guide a change in the identified wearing state. The method may include, in response to identifying that the identified wearing state is the first wearing state, obtaining biometric data through a biometric sensor 171 of the wearable device 101. The method may include obtaining the biometric information based on the obtained biometric data.

The method may include, in response to an input requesting for obtaining biometric information, obtaining biometric data through a biometric sensor 171 of the wearable device 101. The method may include obtaining the biometric information based on the biometric data. The method may include selecting a coefficient corresponding to the identified wearing state among a plurality of coefficients. The method may include adjusting the biometric information based on the selected coefficient.

The method may include identifying that the wearable device 101 is worn by the user, based on that the temperature value changes more than or equal to a reference temperature value within a designated time period.

The method may include obtaining movement data indicating movement of the wearable device 101 through a motion sensor 173 of the wearable device 101. The method may include, in response to that the movement data indicates that the wearable device 101 is moved, determining whether the wearable device 101 is being worn by the user.

The method may include, while the wearable device 101 is worn by the user, identifying a change in the wearing state. The method may include identifying that the changed wearing state is a second wearing state among the plurality of wearing states 601, 603, and 605. The method may include, based on identifying that the changed wearing state is the second wearing state, outputting a notification to guide a change in the wearing state.

A non-transitory computer readable storage medium as described above may store a program including instructions. The instructions may be configured, when executed by at least one processor 120 of the wearable device 101 including a temperature sensor 170 arranged to face skin of a user when the wearable device 101 is worn by the user, to cause the wearable device 101 to identify that the wearable device 101 is worn by the user. The instructions may be configured, when executed by the at least one processor 120, to cause the wearable device 101 to, in response to identifying that the wearable device 101 is worn by the user, identify one wearing state corresponding to temperature data among a plurality of wearing states 601, 603, and 605, the temperature data indicating a temperature value measured through the temperature sensor 170.

The electronic device according to various embodiments may be one of various types of electronic devices. The electronic devices may include, for example, a portable communication device (e.g., a smartphone), a computer device, a portable multimedia device, a portable medical device, a camera, a wearable device, or a home appliance. According to an embodiment of the disclosure, the electronic devices are not limited to those described above.

It should be appreciated that various embodiments of the present disclosure and the terms used therein are not intended to limit the technological features set forth herein to particular embodiments and include various changes, equivalents, or replacements for a corresponding embodiment. With regard to the description of the drawings, similar reference numerals may be used to refer to similar or related elements. It is to be understood that a singular form of a noun corresponding to an item may include one or more of the things unless the relevant context clearly indicates otherwise. As used herein, each of such phrases as "A or B," "at least one of A and B," "at least one of A or B," "A, B, or C," "at least one of A, B, and C," and "at least one of A, B, or C," may include any one of or all possible combinations of the items enumerated together in a corresponding one of the phrases. As used herein, such terms as "1st" and "2nd," or "first" and "second" may be used to simply distinguish a corresponding component from another, and does not limit the components in other aspect (e.g., importance or order). It is to be understood that if an element (e.g., a first element) is referred to, with or without the term "operatively" or "communicatively", as "coupled with," or "connected with" another element (e.g., a second element), it means that the element may be coupled with the other element directly (e.g., wiredly), wirelessly, or via a third element.

As used in connection with various embodiments of the disclosure, the term "module" may include a unit implemented in hardware, software, or firmware, and may interchangeably be used with other terms, for example, "logic," "logic block," "part," or "circuitry". A module may be a single integral component, or a minimum unit or part thereof, adapted to perform one or more functions. For example, according to an embodiment, the module may be implemented in a form of an application-specific integrated circuit (ASIC).

Various embodiments as set forth herein may be implemented as software (e.g., the program 1440) including one or more instructions that are stored in a storage medium (e.g., internal memory 1436 or external memory 1438) that is readable by a machine (e.g., the electronic device 1401). For example, a processor (e.g., the processor 1420) of the machine (e.g., the electronic device 1401) may invoke at least one of the one or more instructions stored in the storage medium, and execute it, with or without using one or more other components under the control of the processor. This allows the machine to be operated to perform at least one function according to the at least one instruction invoked. The one or more instructions may include a code generated by a complier or a code executable by an interpreter. The machine-readable storage medium may be provided in the form of a non-transitory storage medium. Wherein, the term "non-transitory" simply means that the storage medium is a tangible device, and does not include a signal (e.g., an electromagnetic wave), but this term does not differentiate between a case in which data is semi-permanently stored in the storage medium and a case in which the data is temporarily stored in the storage medium.

According to an embodiment, a method according to various embodiments of the disclosure may be included and provided in a computer program product. The computer program product may be traded as a product between a seller and a buyer. The computer program product may be distributed in the form of a machine-readable storage medium (e.g., compact disc read only memory (CD-ROM)), or be distributed (e.g., downloaded or uploaded) online via an application store (e.g., PlayStore^{™}), or between two user devices (e.g., smart phones) directly. If distributed online, at least part of the computer program product may be temporarily generated or at least temporarily stored in the machine-readable storage medium, such as memory of the manufacturer's server, a server of the application store, or a relay server.

According to various embodiments, each component (e.g., a module or a program) of the above-described components may include a single entity or multiple entities, and some of the multiple entities may be separately disposed in different components. According to various embodiments, one or more of the above-described components may be omitted, or one or more other components may be added. Alternatively or additionally, a plurality of components (e.g., modules or programs) may be integrated into a single component. In such a case, according to various embodiments, the integrated component may still perform one or more functions of each of the plurality of components in the same or similar manner as they are performed by a corresponding one of the plurality of components before the integration. According to various embodiments, operations performed by the module, the program, or another component may be carried out sequentially, in parallel, repeatedly, or heuristically, or one or more of the operations may be executed in a different order or omitted, or one or more other operations may be added.

## Claims

1. A wearable device (101), comprising:
a temperature sensor (170) arranged to face skin of a user, when the wearable device (101) is worn by the user,
at least one processor (120), and
memory (130) storing instructions, wherein the instructions are configured, when executed individually or collectively by the at least one processor (120), to cause the wearable device (101) to:
identify that the wearable device (101) is worn by the user, and
in response to identifying that the wearable device (101) is worn by the user, identify one wearing state corresponding to temperature data among a plurality of wearing states (601, 603, 605), the temperature data indicating a temperature value measured through the temperature sensor (170).

2. The wearable device (101) of claim 1, further comprising:
a biometric sensor (171) obtaining biometric data related to the user,
wherein the instructions are configured, when executed individually or collectively by the at least one processor (120), to cause the wearable device (101) to:
in response to an input requesting for obtaining biometric information, identify whether the identified wearing state is a first wearing state for obtaining the biometric data,
in response to identifying that the identified wearing state is different from the first wearing state, output a notification to guide a change in the identified wearing state,
in response to identifying that the identified wearing state is the first wearing state, obtain biometric data through the biometric sensor (171), and
obtain the biometric information based on the obtained biometric data.

3. The wearable device (101) of any one of claims 1 to 2, further comprising:
a biometric sensor (171) obtaining biometric data related to the user,
wherein the instructions are configured, when executed individually or collectively by the at least one processor (120), to cause the wearable device (101) to:
in response to an input requesting for obtaining biometric information, obtain biometric data through the biometric sensor (171),
obtain the biometric information based on the obtained biometric data,
select a coefficient corresponding to the identified wearing state among a plurality of coefficients, and
adjust the biometric information based on the selected coefficient.

4. The wearable device (101) of any one of claims 1 to 3,
wherein the instructions are configured, when executed individually or collectively by the at least one processor (120), to cause the wearable device (101) to:
identify that the wearable device (101) is worn by the user, based on that the temperature value changes more than or equal to a reference temperature value within a designated time period.

5. The wearable device (101) of claim 4, further comprising:
a proximity sensor (172),
wherein the instructions are configured, when executed individually or collectively by the at least one processor (120), to cause the wearable device (101) to:
identify a first state of the wearable device (101) based on a proximity sensing value obtained from the proximity sensor (172),
identify a second state of the wearable device (101) based on that the temperature value changes more than or equal to the reference temperature value within the designated time period, and
identify that the wearable device (101) is worn by the user based on that both the first state and the second state indicate that the wearable device (101) is worn by the user.

6. The wearable device (101) of any one of claims 1 to 5, further comprising:
a motion sensor (173),
wherein the instructions are configured, when executed individually or collectively by the at least one processor (120), to cause the wearable device (101) to:
obtain movement data indicating movement of the wearable device (101) through the motion sensor (173), and
in response to that the movement data indicates that the wearable device (101) is moved, determine whether the wearable device (101) is being worn by the user.

7. The wearable device (101) of any one of claims 1 to 6,
wherein the instructions are configured, when executed individually or collectively by the at least one processor (120), to cause the wearable device (101) to:
while the wearable device (101) is worn by the user, identify a change in the wearing state,
identify that the changed wearing state is a second wearing state among the plurality of wearing states (601, 603, 605), and
based on identifying that the changed wearing state is the second wearing state, output a notification to guide a change in the wearing state.

8. The wearable device (101) of claim 7,
wherein the instructions are configured, when executed individually or collectively by the at least one processor (120), to cause the wearable device (101) to:
after identifying that the changed wearing state is the second wearing state, based on that the second wearing state is maintained more than or equal to a designated time period, output the notification.

9. The wearable device (101) of claim 7, further comprising:
a pressure sensor (175) arranged to face the skin of the user,
wherein the instructions are configured, when executed individually or collectively by the at least one processor (120), to cause the wearable device (101) to:
based on identifying that the changed wearing state is the second wearing state, obtain a pressure signal through the pressure sensor (175), and
based on that a pressure value indicated by the pressure signal exceeds a designated pressure value, output the notification.

10. The wearable device (101) of any one of claims 1 to 9, further comprising:
communication circuitry (190),
wherein the instructions are configured, when executed individually or collectively by the at least one processor (120), to cause the wearable device (101) to:
establish a communication connection with an external electronic device (102) through the communication circuitry (190); and
based on the communication connection, transmit data indicating the identified wearing state to the external electronic device (102) for the external electronic device (102) to output a notification to guide the identified wearing state to the user.

11. The wearable device (101) of any one of claims 1 to 10,
wherein the instructions are configured, when executed individually or collectively by the at least one processor (120), to cause the wearable device (101) to:
identify that the wearable device (101) is worn on a finger corresponding to the temperature data among fingers of the user.

12. The wearable device (101) of claim 11, further comprising:
a motion sensor (173),
wherein the instructions are configured, when executed individually or collectively by the at least one processor (120), to cause the wearable device (101) to:
based on that the wearable device (101) is worn on a first finger among the fingers: obtain movement data indicating movement of the wearable device (101) through the motion sensor (173), and identify a gesture corresponding to a movement path of the wearable device (101) indicated by the movement data, and
based on identifying that the wearable device (101) is worn on a finger other than the first finger among the fingers, output a notification to guide a change in the identified wearing state.

13. The wearable device (101) of any one of claims 1 to 12, further comprising:
a motion sensor (173),
wherein the instructions are configured, when executed individually or collectively by the at least one processor (120), to cause the wearable device (101) to:
obtain movement data indicating movement of the wearable device (101) through the motion sensor (173), and
based on a movement path of the wearable device (101) indicated by the movement data, identify a hand where a finger on which the wearable device (101) is worn is located among both hands of the user.

14. The wearable device (101) of claim 13,
wherein the instructions are configured, when executed individually or collectively by the at least one processor (120), to cause the wearable device (101) to:
obtain a tracking input for tracking a movement path,
in response to the tracking input, determine whether the identified hand is a designated hand,
in response to identifying that the identified hand is the designated hand, obtain movement data for tracking the movement path, and
in response to identifying that the identified hand is different from the designated hand, output a notification to guide a change in the identified wearing state.

15. A method performed by a wearable device (101) including a temperature sensor (170) arranged to face skin of a user, when the wearable device (101) is worn by the user, comprising:
identifying that the wearable device (101) is worn by the user, and
in response to identifying that the wearable device (101) is worn by the user, identifying one wearing state corresponding to temperature data among a plurality of wearing states (601, 603, 605), the temperature data indicating a temperature value measured through the temperature sensor (170).
